# EUROPEAN PATENT APPLICATION

(11) **EP 3 236 265 A1**
(43) Date of publication of application: **25.10.2017**
(21) Application number: 16166744.9
(22) Date of filing: 22.04.2016
(51) Int. Cl.: G01N 33/92

(54) **METHOD FOR THE DIAGNOSIS OF CYSTIC FIBROSIS**

(71) Applicant: Centogene IP GmbH, 18057 Rostock (DE)
(72) Inventor: The designation of the inventor has not yet been filed
(74) Representative: Bohmann, Armin K.

(57) **Abstract**

The present invention is related to a method for diagnosing cystic fibrosis in a subject comprising
a step a), wherein the step a) comprises detecting a biomarker in a sample from the subject, wherein the sample is a sample selected from the group consisting of blood, blood plasma, blood serum and a blood product.

## Description

The present invention is related to a method for diagnosing cystic fibrosis in a subject, a method for determining the course of cystic fibrosis in a subject, a method for determining the effectiveness of at least one treatment applied to a subject being positively tested for suffering from or being at risk of suffering from cystic fibrosis, a method for determining the effectiveness of a compound for the treatment of cystic fibrosis, use of mass spectrometric analysis for the detection of a biomarker, use of a biomarker for the diagnosis of cystic fibrosis, use of an internal standard to quantify a biomarker present in a sample from the subject, and a kit for determining the presence of a biomarker in a sample from a subject.

### Background

Cystic fibrosis (CF), also known as mucoviscidosis, is a genetic disorder that affects mostly the lungs but also the pancreas, liver, kidneys, and intestine. Symptoms include difficulty breathing and coughing up mucus as a result of frequent lung infections, sinus infections, poor growth, fatty stool, clubbing of the finger and toes, and infertility in males. Both mild and severe cases were described.

CF is inherited in an autosomal recessive manner. It is caused by the presence of mutations in both copies of the gene for the cystic fibrosis transmembrane conductance regulator (CFTR) protein. Although cystic fibrosis occurs in all races, it is most common in white people of Northern European ancestry. Ceramide accumulates in the lungs of CF patients and in individuals with other chronic lung diseases such as, e.g., emphysema.

The mechanism(s) underlying ceramide accumulation in CF are complex, but in part are due to alterations in lung pH that favor high activity of the ceramide producing enzyme, acid sphingomyelinase (ASM), and low activity of the ceramide clearing enzyme, acid ceramidase (AC). Previous studies suggested that a defect in the cystic fibrosis transmembrane regulator protein (CFTR) may itself be responsible for high ceramide levels in CF patients - PDZ interacting domain of the CFTR protein localizes the CFTR protein to lipid rafts in the cell membrane, and this localization suppresses cellular ceramide levels in healthy individuals, but not in patients with CF, due potentially to a defect in the PDZ domain. High lung ceramide levels amplify the inflammatory pathology in CF patients and directly drive susceptibility to pathogens. Healthy individuals maintain low levels of ceramide in their lung epithelial cells through the homeostatic-like regulation of the enzymes ASM and AC and their activities. In CF lungs ceramide levels become high due, in part, to alterations of these enzymatic activities, typically high ASM and low AC. In addition, mutations in CFTR may alter delivery of the protein to the membrane rafts, which also contributes to ceramide elevation.

While there are no cures for cystic fibrosis, there are several treatment methods. The cornerstones of cystic fibrosis management are proactive treatment of airway infection, and encouragement of good nutrition and an active lifestyle. Pulmonary rehabilitation as a management of cystic fibrosis continues throughout a person's life, and is aimed at maximizing organ function, and therefore quality of life. At best, current treatments delay the decline in organ function taking into consideration the wide variation in disease symptoms. Targets for therapy are the lungs, gastrointestinal tract including pancreatic enzyme supplements, the reproductive organs including assisted reproductive technology (ART) and psychological support. Still, the most consistent aspect of therapy in cystic fibrosis is limiting and treating the lung damage caused by thick mucus and infection, with the goal of maintaining quality of life. Intravenous, inhaled, and oral antibiotics are used to treat chronic and acute infections. Mechanical devices and inhalation medications are used to alter and clear the thickened mucus.

Cystic fibrosis may be diagnosed by many different methods including newborn screening, sweat testing, and genetic testing. The newborn screen initially measures for raised blood concentration of immunoreactive trypsinogen. Infants with an abnormal newborn screen typically undergo a sweat test to confirm the CF diagnosis. Trypsinogen levels can be increased in individuals who have a single mutated copy of the CFTR gene. Such individuals are also referred to as carriers. Most individuals are diagnosed after symptoms such as, e.g., sinopulmonary disease and gastrointestinal (GI) manifestations which prompt an evaluation for cystic fibrosis. The most commonly used form of testing is the sweat test. Sweat-testing involves application of a medication that stimulates sweating such as pilocarpine. People with CF have increased amounts of sodium and chloride in their sweat. In contrast, people with CF have less thiocyanate and hypothiocyanite in their saliva and mucus. CF can also be diagnosed by identification of mutations in the CFTR gene. Several thousand mutations of the CFTR gene are known, whereas in commercial testing only the most frequent ones such as ΔF508 are detected.

The above mentioned laboratory tests, i.e. immunoreactive trypsinogen and sweat test, are nonspecific, time consuming and demand high levels or resources ( biological sample, time and laboratory materials). In light thereof, the problem underlying the present invention is the provision of a means for the diagnosis of cystic fibrosis in a high throughput manner which is suitable for mass screenings with low biological sample consumption. A further problem underlying the present invention is the provision of a means for the diagnosis of cystic fibrosis making use of a biomarker, preferably making use of a biomarker contained in blood or a blood product from the subject to be diagnosed.

These and other problems are solved by the subject matter of the attached independent claims. Preferred embodiments may be taken from the attached dependent claims.

These and other problems are equally solved by the subject matter of the following embodiments 1 to 137 and further embodiments disclosed herein. It is to be taken into consideration that the subject matter of embodiments 1, 30, 52, 79, 99, 125, 131, 135 and 137 is each also referred to as aspect of the present invention.
Embodiment 1. A method for diagnosing cystic fibrosis in a subject comprising a step a), wherein the step a) comprises detecting a biomarker in a sample from the subject.
Embodiment 2. The method according to embodiment 1, wherein the method comprises
   a step b) wherein the step b) comprises determining a level of the biomarker present in the sample.
Embodiment 3. The method according to any one of embodiments 1 or 2, wherein the level of the biomarker is indicative whether or not the subject is suffering from cystic fibrosis or whether or not the subject is at risk of suffering from cystic fibrosis.
Embodiment 4. The method according to any one of embodiments 1 to 3, wherein the sample from the subject is a sample from a subject who has previously been treated for cystic fibrosis or a sample from a subject who has previously been diagnosed for cystic fibrosis.
Embodiment 5. The method according to any one of embodiments 1 to 3, wherein the sample from the subject is a sample from a subject who has not previously been treated for cystic fibrosis or a sample from a subject who has not been previously diagnosed for cystic fibrosis.
Embodiment 6. The method according to any one of embodiments 1 to 5, wherein the method comprises a step c), wherein the step c) comprises applying, maintaining, reducing, elevating or not applying a therapy based on whether the subject is suffering from cystic fibrosis or is at risk of suffering from cystic fibrosis.
Embodiment 7. The method according to embodiment 6, wherein the method comprises a step d), wherein the step d) comprises detecting the biomarker in a sample from the subject after a therapy has been applied, maintained, reduced, elevated or not applied in step c).
Embodiment 8. The method according to any one of embodiments 6 to 7, wherein the method comprises a step e), wherein the step e) comprises determining a level of the biomarker in the sample from the subject after a therapy has been applied, maintained, reduced, elevated or not applied in step c).
Embodiment 9. The method according to embodiment 8, wherein the method comprises a step f), wherein the step f) comprises determining whether the level of the biomarker determined in step b) is lower than the level of the biomarker determined in step e).
Embodiment 10. The method according to embodiment 9, wherein the method comprises a step g), wherein the step g) comprises applying, maintaining, reducing, elevating or not applying a therapy based on step f).
Embodiment 11. The method according to any one of embodiments 1 to 10, wherein the biomarker is one selected from the group consisting of C26 ceramide, a medium ceramide and a long ceramide, wherein a medium ceramide is a ceramide of formula (2) 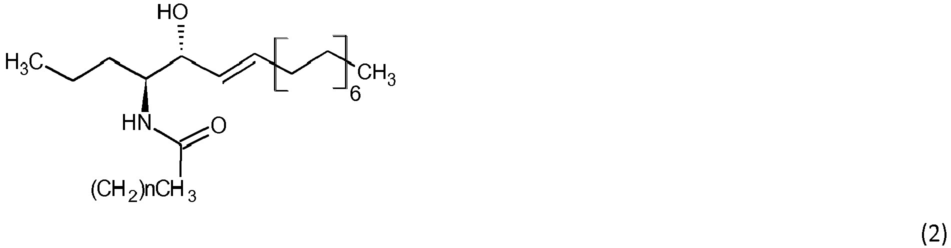 wherein n is any integer from: 8, 10, 12, 14 and 16 and
   a long ceramide is a ceramide of formula (2) 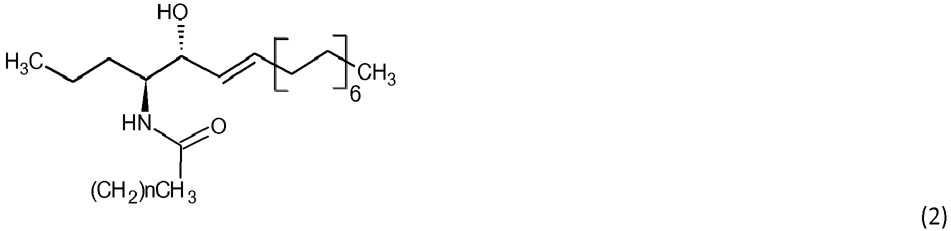 wherein n is any integer from 18, 20, 22, 24, 26 and 28.
Embodiment 12. The method according to embodiment 11, wherein the biomarker is C26 ceramide.
Embodiment 13. The method according to any one of embodiments 11 to 12, wherein the biomarker is cis-C26 ceramide.
Embodiment 14. The method according to any one of embodiments 11 to 12, wherein the biomarker is total C26 ceramide, preferably total C26 ceramide is cis-C26 ceramide plus trans-C26 ceramide.
Embodiment 15. The method according to any one of embodiments 11 to 14, wherein the method comprises determining the level of the biomarker in a or the sample, preferably the level of cis-C26 ceramide or the level of total C26 ceramide, wherein, preferably as used herein for each and any aspect of the invention, the level of total C26 ceramide is the sum of the level of cis-C26 ceramide and the level of trans-C26 ceramide.
Embodiment 16. The method according to any one of embodiments 1 to 15, wherein the biomarker is detected by means of immunoassay, mass spectrometric analysis, affinity chromatography, affinity - mass spectrometry, biochip array, functional nucleic acids and/or a fluorescent a derivative of the biomarker, wherein preferably the derivative of the biomarker is a labelled derivative of the biomarker, wherein more preferably the labeled derivative of the biomarker is selected from the group comprising a fluorescent derivative of the biomarker, a biologically labelled derivative of the biomarker, a physically labelled derivative of the biomarker and a chemically labelled derivative of the biomarker.
Embodiment 17. The method according to embodiment 16, wherein the biomarker is detected by means of mass spectrometric analysis.
Embodiment 18. The method according to embodiment 17, wherein mass spectrometric analysis is selected from the group comprising SELDI MS, MALDI MS, ESI MS, DESI MS and ion mobility MS.
Embodiment 19. The method according to any one of embodiments 16 to 18, wherein mass spectrometric analysis uses an analyzer selected from the group comprising ToF, QToF, ion trap, Triple Quad, orbitrap, ion mobility and any combination thereof.
Embodiment 20. The method according to any one of embodiments 16 to 19, wherein the mass spectrometric analysis comprises or uses MS/MS, MRM, SRM or any combination thereof, preferably MS/MS, MS/MRM or MS/SRM.
Embodiment 21. The method according to any one of embodiments 1 to 20, wherein the method comprises protein precipitation from the sample and/or extraction of the biomarker from the sample.
Embodiment 22. The method according to any one of embodiments 1 to 21, wherein the method comprises protein precipitation from the sample.
Embodiment 23. The method according to any one of embodiments 21 to 22, wherein the method comprises HPLC.
Embodiment 24. The method according to embodiment 23, wherein the method comprises
   a) protein precipitation from the sample and HPLC;
   b) extraction of the biomarker from the sample and HPLC; and/or
   c) protein precipitation from the sample, extraction of the biomarker from the sample and HPLC.
Embodiment 25. The method according to any one of embodiments 21 to 22, wherein the method further comprises at least one from the group consisting of HPLC, MS/MS, MRM and MS/MS-MRM.
Embodiment 26. The method according to embodiment 25, wherein the method comprises
   a) protein precipitation from the sample, HPLC and MS/MS or MRM;
   b) protein precipitation from the sample, HPLC and MS/MS.MRM;
   c) extraction of the biomarker from the sample, HPLC and MS/MS or MRM;
   d) extraction of the biomarker from the sample, HPLC and MS/MS.MRM;
   e) protein precipitation and extraction of the biomarker from the sample, HPLC and MS/MS or MRM; and/or
   f) protein precipitation and extraction of the biomarker from the sample, HPLC and MS/MS.MRM.
Embodiment 27. The method according to any one of embodiments 1 to 26, wherein the subject is a human.
Embodiment 28. The method according to embodiment 27, wherein the subject is a human subject suffering from or being at risk of suffering from cystic fibrosis.
Embodiment 29. The method according to any one of embodiments 7 to 28, wherein step d) comprising detecting the biomarker in a sample comprises subjecting the sample to a protein precipitation and/or biomarker extraction step, precipitating protein and/or extraction biomarker from the sample, providing a supernatant of the sample, subjecting the supernatant of the sample to HPLC and MS/MS and determining the level of the biomarker present in the supernatant and/or extract of the sample.
Embodiment 30. A method for diagnosing cystic fibrosis in a subject, wherein the method comprises the following steps:
   i) adding an internal standard to a sample from the subject, wherein the sample from the subject is selected from the group comprising plasma, serum, blood and a blood product, whereby the sample is preferably liquid blood or a dried blood spot;
   ii) optionally mixing the sample with the internal standard;
   iii) subjecting the sample to a protein precipitation and/or a biomarker extraction step, whereby protein from the sample is precipitated and/or the biomarker is extracted and a first supernatant of the sample is provided;
   iv) optionally subjecting the first supernatant of the sample or at least a part thereof to a first separation step which provides a second supernatant, whereby preferably the first separation step is a step of centrifugation;
   v) subjecting the first supernatant and/or the second supernatant, or at least a part thereof, to a second separation step, wherein the second separation step comprises injecting at least a part of the first supernatant and/or at least a part of the second supernatant into an HPLC-MS/MS system and using an HPLC column with a gradient from acidic water to acetonitrile/acetone; wherein the HPLC column is preferably an HPLC column selected from the group comprising a C8 RP-HPLC column and a C18 RP-HPLC column, and wherein the second separation step provides a separated sample;
   vi) subjecting the separated sample to MS/MS, wherein MS/MS comprises in source-fragmentation, collision induced dissociation, electron-capture dissociation, electron-transfer dissociation, negative electron dissociation, electron-detachment dissociation, charge transfer dissociation and/or photo dissociation,
   and wherein the method comprises
   a step a), wherein the step a) comprises detecting a biomarker in a sample from the subject,
   and optionally
   a step b), wherein the step b) comprises determining a level of the biomarker present in the sample, and
   wherein the method is otherwise preferably a method according to any one of embodiments 1 to 29.
Embodiment 31. The method according to embodiment 30, wherein the internal standard is selected from the group comprising N-lauroyl sphingosine, lyso-Gb2, a C17 ceramide, a C19 ceramide, a C21 ceramide, a C23 ceramide and a C25 ceramide.
Embodiment 32. The method according to any one of embodiments 1 to 31, wherein step b), step c) and/or step e) comprises comparing the level of the biomarker in the sample with a cut-off value.
Embodiment 33. The method according to embodiment 32, wherein if the level of the biomarker in the sample from the subject is higher than the cut-off value this is indicative that the subject is suffering from cystic fibrosis or is at risk of suffering from cystic fibrosis.
Embodiment 34. The method according to embodiment 32, wherein if the level of the biomarker in the sample from the subject is lower than the cut-off value this is indicative that the subject is not suffering from or is not at risk of suffering from cystic fibrosis.
Embodiment 35. The method according to any one of embodiments 32 to 34, wherein the cut-off value is selected such that a or the sensitivity for diagnosing cystic fibrosis in a subject is from about 95% to 100%, preferably from about 98.5% to 100% more preferably 99.5% to 100%.
Embodiment 36. The method according to any one of embodiments 1 to 35, wherein step b) and/or step c) and/or step e) comprises that a level of the biomarker in said subject is compared to a level of the biomarker detected in a sample from a control sample;
Embodiment 37. The method according to embodiment 36, wherein the control sample is a sample from a subject not having cystic fibrosis.
Embodiment 38. The method according to any one of embodiments 36 to 37, wherein if the level of the biomarker in the sample from the subject is higher than the level of the biomarker in the control sample this is indicative that the subject is suffering from and/or is at risk of suffering from cystic fibrosis.
Embodiment 39. The method according to any one of embodiments 1 to 38, wherein the sample from the subject is selected from the group comprising blood, a blood product, urine, saliva, cerebrospinal fluid, stool, tissue sample and lymph.
Embodiment 40. The method according to embodiment 39, wherein the sample from the sample from the subject is selected from the group comprising blood and a blood product.
Embodiment 41. The method according to any one of embodiments 39 to 40, wherein the blood product is dried blood spots (DBS).
Embodiment 42. The method according to any one of embodiments 1 to 41, preferably 41, wherein the method has a limit of detection for C26 ceramide of 0.1 nmol/L blood, wherein, preferably, the biomarker is detected by means of mass spectrometry.
Embodiment 43. The method according to any one of embodiments 32 to 42, wherein the method is for the diagnosis of cystic fibrosis and wherein the cut-off value is 69 nmol/L, preferably 69.1 nmol/L, in case the biomarker is total C26 ceramide and wherein the cut-off value is 27.7 nmol/L in case the biomarker is cis-C26 ceramide.
Embodiment 44. The method of embodiment 43, wherein the sample from the subject is dried blood spots (DBS).
Embodiment 45. The method according to any one of embodiments 1 to 44, wherein the subject has been previously treated for cystic fibrosis and/or wherein the subject has been previously diagnosed for cystic fibrosis.
Embodiment 46. The method according to any one of embodiments 1 to 45, wherein the subject has not been previously treated for cystic fibrosis and/or wherein the subject has not been previously diagnosed for cystic fibrosis.
Embodiment 47. The method according to any one of embodiments 30 to 46, wherein the method comprises a step c), wherein the step c) comprises applying, maintaining, reducing, elevating or not applying a therapy based on whether the subject is suffering from cystic fibrosis or is at risk of suffering from cystic fibrosis.
Embodiment 48. The method according to any one of embodiments 30 to 46, wherein the method comprises a step d), wherein the step d) comprises detecting the biomarker in a sample from the subject after a therapy has been applied, maintained, reduced, elevated or not applied in step c).
Embodiment 49. The method according to any one of embodiments 30 to 48, wherein the method comprises a step e), wherein the step e) comprises determining a level of the biomarker in the sample from the subject after a therapy has been applied, maintained, reduced, elevated or not applied in step c).
Embodiment 50. The method according to any one of embodiments 30 to 49, wherein the method comprises a step f), wherein the step f) comprises determining whether the level of the biomarker determined in step b) is lower than the level of the biomarker determined in step f).
Embodiment 51. The method according to any embodiment 50, wherein the method comprises
   a step g), wherein the step g) comprises applying, maintaining, reducing, elevating or not applying a therapy based on step f).
Embodiment 52. A method for determining the effectiveness of at least one treatment applied to a subject being positively tested for suffering from or being at risk of suffering from cystic fibrosis comprising
   a step a), wherein the step a) comprises determining at several points in time a level of a biomarker in a sample from the subject, wherein the biomarker is preferably one selected from the group consisting of C26 ceramide, a medium ceramide and a long ceramide, wherein a medium ceramide is a ceramide of formula (2) 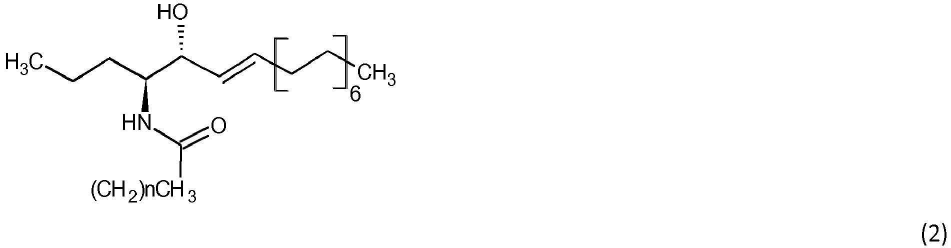 wherein n is any integer from: 8, 10, 12, 14 and 16 and
   a long ceramide is a ceramide of formula (2) 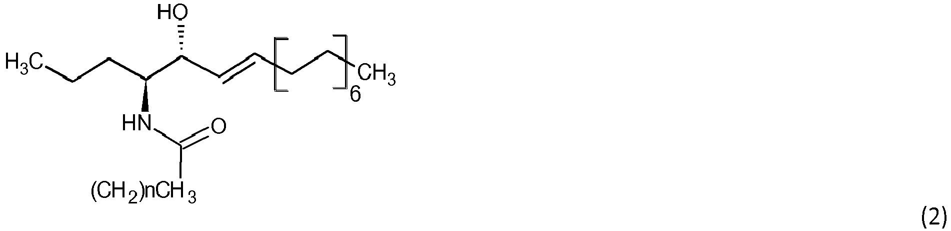 wherein n is any integer from 18, 20, 22, 24, 26 and 28.
Embodiment 53. The method according to embodiment 52, wherein the biomarker is C26 ceramide.
Embodiment 54. The method according to any one of embodiments 52 to 53, wherein the biomarker is cis-C26 ceramide.
Embodiment 55. The method according to any one of embodiments 52 to 53, wherein the biomarker is total C26 ceramide, preferably total C26 ceramide is cis-C26 ceramide and trans-C26 ceramide.
Embodiment 56. The method according to any one of embodiments 52 to 55, wherein the method comprises determining the level of the biomarker in a or the sample, preferably the level of cis-C26 ceramide or the level of total C26 ceramide, wherein, preferably as used herein for each and any aspect of the invention, the level of total C26 ceramide is the sum of the level of cis-C26 ceramide and the level of trans-C26 ceramide.
Embodiment 57. The method according to any one of embodiments 52 to 56, wherein the subject has been previously treated for cystic fibrosis or diagnosed for cystic fibrosis.
Embodiment 58. The method according to any one of embodiments 52 to 56, wherein the subject has not been previously treated for cystic fibrosis or wherein the subject has not been previously diagnosed for cystic fibrosis.
Embodiment 59. The method according to any one of embodiments 52 to 58, wherein the method comprises a step b), wherein the step b) comprises applying, maintaining, reducing, elevating or not applying at least one treatment applied to the subject based on the decrease in the level of the biomarker as determined in step a)
Embodiment 60. The method according to any one of embodiments 52 to 59, wherein the method comprises a step c), wherein the step c) comprises detecting the biomarker in the sample from the subject, wherein the sample has been taken prior to the beginning of the treatment after applying, maintaining, reducing, elevating or not applying at least one treatment in step b) and, optionally determining a level of a biomarker present in a sample from the subject,
Embodiment 61. The method according to any one of embodiments 1 to 60, wherein the treatment and/or therapy is selected from the group comprising an inhibitor of acid sphinomyelinase (ASM) and enzyme replacement therapy, whereby preferably the inhibitor is selected from the group comprising Amitriptyline (Elavil), Clomipramine (Anafranil), Desipramine (Norpramin), Doxepine (Sinequan), Imipramine (Tofranil), Lofepramine (Gamanil), Nortrityline (Aventyl), Protriptyline (Vivactil) and Trimipramine (Surmontil) and whereby preferably the enzyme of the enzyme replacement therapy is acidic deramindase.
Embodiment 62. The method according to any one of embodiments 52 to 60, wherein the method comprises a step d), wherein the step d) comprises determining whether the level of the biomarker determined in step a) is lower than the level of the biomarker determined in step c);
Embodiment 63. The method according to embodiment 62, wherein the method comprises
   a step e), wherein step e) comprises applying, maintaining, reducing, elevating or not applying at least one treatment applied to the subject based on step d).
Embodiment 64. The method according to any one of embodiments 30 to 63, wherein the biomarker is detected by means of immunoassay, mass spectrometric analysis, affinity chromatography, affinity - mass spectrometry, biochip array, functional nucleic acids and/or a fluorescent a derivative of the biomarker, wherein preferably the derivative of the biomarker is a labelled derivative of the biomarker, wherein more preferably the labeled derivative of the biomarker is selected from the group comprising a fluorescent derivative of the biomarker, a biologically labelled derivative of the biomarker, a physically labelled derivative of the biomarker and a chemically labelled derivative of the biomarker.
Embodiment 65. The method according to embodiment 64, wherein the biomarker is detected by means of mass spectrometric analysis.
Embodiment 66. The method according to embodiment 65, wherein the biomarker is detected by means of mass spectrometric analysis.
Embodiment 67. The method according to embodiment 65, wherein mass spectrometric analysis is selected from the group comprising SELDI MS, MALDI MS, ESI MS, DESI MS and ion mobility MS.
Embodiment 68. The method according to any one of embodiments 65 to 67, wherein mass spectrometric analysis uses an analyzer selected from the group comprising ToF, QToF, ion trap, Triple Quad, orbitrap, ion mobility and any combination thereof.
Embodiment 69. The method according to any one of embodiments 65 to 68, wherein the mass spectrometric analysis comprises or uses MS/MS, MRM, SRM or any combination thereof, preferably MS/MS, MS/MRM or MS/SRM.
Embodiment 70. The method according to any one of embodiments 52 to 69, wherein the method comprises protein precipitation from the sample and/or extraction of the biomarker from the sample.
Embodiment 71. The method according to any one of embodiments 52 to 70, wherein the method comprises protein precipitation from the sample.
Embodiment 72. The method according to any one of embodiments 70 to 71, wherein the method comprises HPLC.
Embodiment 73. The method according to embodiment 72, wherein the method comprises
   a) protein precipitation from the sample and HPLC;
   b) extraction of the biomarker from the sample and HPLC; and/or
   c) protein precipitation from the sample, extraction of the biomarker from the sample and HPLC.
Embodiment 74. The method according to any one of embodiments 70 to 71, wherein the method further comprises at least one from the group consisting of HPLC, MS/MS, MRM and MS/MS-MRM.
Embodiment 75. The method according to embodiment 74, wherein the method comprises
   a) protein precipitation from the sample, HPLC and MS/MS or MRM;
   b) protein precipitation from the sample, HPLC and MS/MS.MRM;
   c) extraction of the biomarker from the sample, HPLC and MS/MS or MRM;
   d) extraction of the biomarker from the sample, HPLC and MS/MS.MRM;
   e) protein precipitation and extraction of the biomarker from the sample, HPLC and MS/MS or MRM; and/or
   f) protein precipitation and extraction of the biomarker from the sample, HPLC and MS/MS.MRM.
Embodiment 76. The method according to any one of embodiments 52 to 75, wherein the subject is a human.
Embodiment 77. The method according to embodiment 76, wherein the subject is a subject suffering from or being at risk of suffering from cystic fibrosis.
Embodiment 78. The method according to any one of embodiments 52 to 77, wherein the step of detecting the biomarker in the sample from the subject comprises precipitating protein and/or biomarker extraction from the sample from the subject, wherein precipitating protein and/or extracting biomarker from the sample provides a supernatant of the sample; subjecting a volume of the supernatant to HPLC and MS/MS and determining the level of the biomarker that is present in the sample from the subject.
Embodiment 79. A method of determining the effectiveness of a compound for the treatment of cystic fibrosis, wherein the method comprises the following steps:
   a) determining a level of a biomarker in a sample from a subject having cystic fibrosis;
   b) administering to said subject said compound;
   c) determining the level of the biomarker in a sample from the subject after the compound has been administered to the subject; and
   d) determining whether the level of the biomarker determined in step c) is lower than the level of the biomarker determined in step a);
   wherein if a level of the biomarker determined in step c) is lower than the level of the biomarker determined in step a) this indicates the effectiveness of said compound.
80. The method of embodiment 79, wherein the biomarker is one selected from the group consisting of C26 ceramide, a medium ceramide and a long ceramide, wherein a medium ceramide is a ceramide of formula (2) 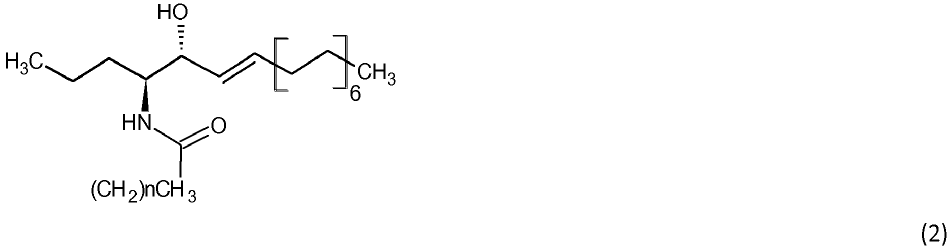 wherein n is any integer from: 8, 10, 12, 14 and 16 and
   a long ceramide is a ceramide of formula (2) 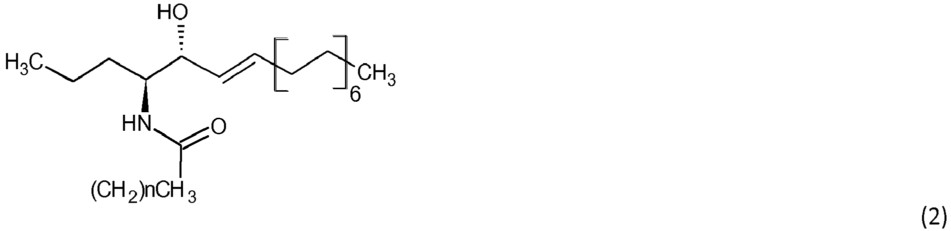 wherein n is any integer from 18, 20, 22, 24, 26 and 28.
Embodiment 81. The method according to embodiment 80, wherein the biomarker is C26 ceramide.
Embodiment 82. The method according to any one of embodiments 80 to 81, wherein the biomarker is cis-C26 ceramide.
Embodiment 83. The method according to any one of embodiments 80 to 81, wherein the biomarker is total C26 ceramide, preferably total C26 ceramide is cis-C26 ceramide and trans-C26 ceramide.
Embodiment 84. The method according to any one of embodiments 80 to 73, wherein the method comprises determining the level of the biomarker in a or the sample, preferably the level of cis-C26 ceramide or the level of total C26 ceramide, wherein, preferably as used herein for each and any aspect of the invention, the level of total C26 ceramide is the sum of the level of cis-C26 ceramide and the level of trans-C26 ceramide.
Embodiment 85. The method according to embodiment 64, wherein the method comprises determining a level of the biomarker in a control sample.
Embodiment 86. The method according to any one of embodiments 79 to 55, wherein the biomarker is detected by means of immunoassay, mass spectrometric analysis, biochip array, functional nucleic acids and/or a fluorescent derivative of the biomarker.
Embodiment 87. The method according to embodiment 86, wherein the biomarker is detected by means of mass spectrometric analysis.
Embodiment 88. The method according to embodiment 87, wherein mass spectrometric analysis is selected from the group comprising SELDI MS, MALDI MS, ESI MS, DESI MS and ion mobility MS.
Embodiment 89. The method according to any one of embodiments 86 to 88, wherein mass spectrometric analysis uses an analyzer selected from the group comprising ToF, QToF, ion trap, Triple Quad, orbitrap, ion mobility and any combination thereof.
Embodiment 90. The method according to any one of embodiments 86 to 89, wherein the mass spectrometric analysis comprises or uses MS/MS, MRM, SRM or any combination thereof, preferably MS/MS, MS/MRM or MS/SRM.
Embodiment 91. The method according to any one of embodiments 79 to 90, wherein the method comprises protein precipitation from the sample and/or extraction of the biomarker from the sample.
Embodiment 92. The method according to any one of embodiments 79 to 91, wherein the method comprises protein precipitation from the sample.
Embodiment 93. The method according to any one of embodiments 91 to 92, wherein the method comprises HPLC.
Embodiment 94. The method according to embodiment 93, wherein the method comprises
   a) protein precipitation from the sample and HPLC;
   b) extraction of the biomarker from the sample and HPLC; and/or
   c) protein precipitation from the sample, extraction of the biomarker from the sample and HPLC.
Embodiment 95. The method according to any one of embodiments 91 to 92, wherein the method further comprises at least one from the group consisting of HPLC, MS/MS, MRM and MS/MS-MRM.
Embodiment 96. The method according to embodiment 95, wherein the method comprises
   a) protein precipitation from the sample, HPLC and MS/MS or MRM;
   b) protein precipitation from the sample, HPLC and MS/MS.MRM;
   c) extraction of the biomarker from the sample, HPLC and MS/MS or MRM;
   d) extraction of the biomarker from the sample, HPLC and MS/MS.MRM;
   e) protein precipitation and extraction of the biomarker from the sample, HPLC and MS/MS or MRM; and/or
   f) protein precipitation and extraction of the biomarker from the sample, HPLC and MS/MS.MRM.
Embodiment 97. The method according to any one of embodiments 79 to 96, wherein the subject is a human.
Embodiment 98. The method according to embodiment 97, wherein the subject is a subject suffering from or being at risk of suffering from cystic fibrosis.
Embodiment 99. A method for determining the course of cystic fibrosis in a subject comprising the step of
   a) determining at several points in time a level of a biomarker present in a sample from the subject.
Embodiment 100. The method according to embodiment 99, wherein the subject has been previously treated or diagnosed for cystic fibrosis.
Embodiment 101. The method according to embodiment 99, wherein the subject has not been previously treated or wherein the subject has not been previously diagnosed for cystic fibrosis.
Embodiment 102. The method according to any one of embodiments 99 to 101, further comprising a step of
   b) applying, maintaining, reducing, elevating or not applying a therapy based on the diagnosis of whether the subject is suffering from or for being at risk for developing cystic fibrosis.
Embodiment 103. The method according to any one of embodiments 99 to 102, further comprising a step of
   c) detecting the biomarker in a sample from the subject after applying, maintaining, reducing, elevating or not applying a therapy in a step of b).
Embodiment 104. The method according to any one of embodiments 99 to 103, further comprising a step of
   d) determining a level of the biomarker in the sample from the subject after applying, maintaining, reducing, elevating or not applying a therapy in a step of b).
Embodiment 105. The method according to any one of embodiments 99 to 104, further comprising the steps of
   e) determining whether the level of the biomarker determined in step a) is lower than the level of the biomarker determined in step d);
Embodiment 106. The method according to any embodiment 105, further comprising the step of
   f) applying, maintaining, reducing, elevating or not applying a therapy based on the step of e).
Embodiment 107. The method according to any one of embodiments 99 to 106, wherein the biomarker is one selected from the group consisting of C26 ceramide, a medium ceramide and a long ceramide, wherein a medium ceramide is a ceramide of formula (2) 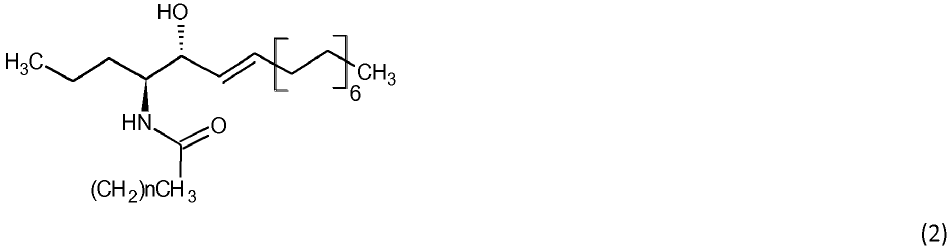 wherein n is any integer from: 8, 10, 12, 14 and 16 and
   a long ceramide is a ceramide of formula (2) 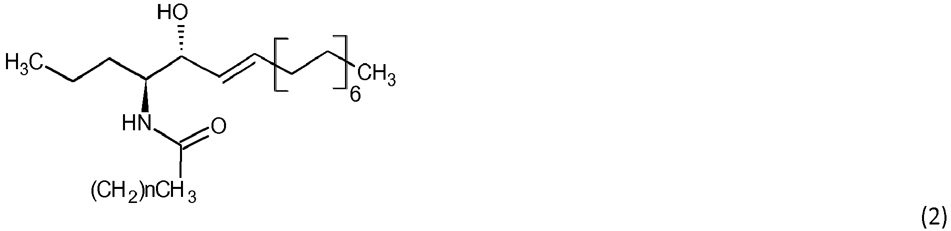 wherein n is any integer from 18, 20, 22, 24, 26 and 28.
Embodiment 108. The method according to embodiment 107, wherein the biomarker is C26 ceramide.
Embodiment 109. The method according to any one of embodiments 107 to 108, wherein the biomarker is cis-C26 ceramide.
Embodiment 110. The method according to any one of embodiments 107 to 108, wherein the biomarker is total C26 ceramide, preferably total C26 ceramide is cis-C26 ceramide and trans-C26 ceramide.
Embodiment 111. The method according to any one of embodiments 107 to 110, wherein the method comprises determining the level of the biomarker in a or the sample, preferably the level of cis-C26 ceramide or the level of total C26 ceramide, wherein, preferably as used herein for each and any aspect of the invention, the level of total C26 ceramide is the sum of the level of cis-C26 ceramide and the level of trans-C26 ceramide.
Embodiment 112. The method according to any one of embodiments 99 to 111, wherein the biomarker is detected by means of immunoassay, mass spectrometric analysis, biochip array, functional nucleic acids and/or a fluorescent derivative of the biomarker.
Embodiment 113. The method according to embodiment 112, wherein the biomarker is detected by means of mass spectrometric analysis.
Embodiment 114. The method according to embodiment 113, wherein mass spectrometric analysis is selected from the group comprising SELDI MS, MALDI MS, ESI MS, DESI MS and ion mobility MS.
Embodiment 115. The method according to any one of embodiments 112 to 114, wherein mass spectrometric analysis uses an analyzer selected from the group comprising ToF, QToF, ion trap, Triple Quad, orbitrap, ion mobility and any combination thereof.
Embodiment 116. The method according to any one of embodiments 112 to 115, wherein the mass spectrometric analysis comprises or uses MS/MS, MRM, SRM or any combination thereof, preferably MS/MS, MS/MRM or MS/SRM.
Embodiment 117. The method according to any one of embodiments 99 to 116, wherein the method comprises protein precipitation from the sample and/or extraction of the biomarker from the sample.
Embodiment 118. The method according to any one of embodiments 99 to 117, wherein the method comprises protein precipitation from the sample.
Embodiment 119. The method according to any one of embodiments 117 to 118, wherein the method comprises HPLC.
Embodiment 120. The method according to embodiment 119, wherein the method comprises
   a) protein precipitation from the sample and HPLC;
   b) extraction of the biomarker from the sample and HPLC; and/or
   c) protein precipitation from the sample, extraction of the biomarker from the sample and HPLC.
Embodiment 121. The method according to any one of embodiments 117 to 118, wherein the method further comprises at least one from the group consisting of HPLC, MS/MS, MRM and MS/MS-MRM.
Embodiment 122. The method according to embodiment 121, wherein the method comprises
   a) protein precipitation from the sample, HPLC and MS/MS or MRM;
   b) protein precipitation from the sample, HPLC and MS/MS.MRM;
   c) extraction of the biomarker from the sample, HPLC and MS/MS or MRM;
   d) extraction of the biomarker from the sample, HPLC and MS/MS.MRM;
   e) protein precipitation and extraction of the biomarker from the sample, HPLC and MS/MS or MRM; and/or
   f) protein precipitation and extraction of the biomarker from the sample, HPLC and MS/MS.MRM.
Embodiment 123. The method according to any one of embodiments 9 to 122, wherein the subject is a human.
Embodiment 124. The method according to embodiment 123, wherein the subject is a subject suffering from or being at risk of suffering from cystic fibrosis.
Embodiment 125. Use of mass spectrometric analysis for the detection of a biomarker, wherein the biomarker is selected from the group consisting of C26 ceramide, a medium ceramide and a long ceramide, wherein a medium ceramide is a ceramide of formula (2) 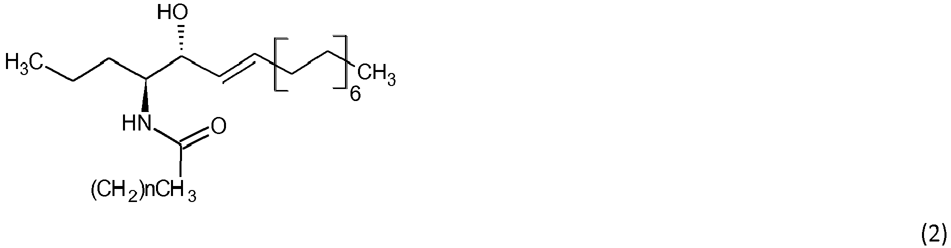 wherein n is any integer from: 8, 10, 12, 14 and 16 and
   a long ceramide is a ceramide of formula (2) 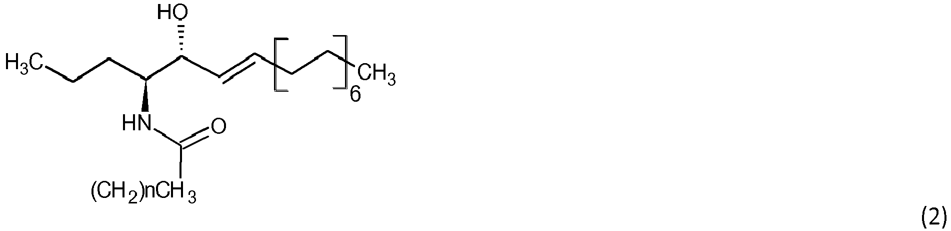 wherein n is any integer from 18, 20, 22, 24, 26 and 28.
Embodiment 126. Use according to embodiment 125, wherein the biomarker is C26 ceramide.
Embodiment 127. Use according to any one of embodiments 125 to 126, wherein the biomarker is cis-C26 ceramide.
Embodiment 128. Use according to any one of embodiments 125 to 126, wherein the biomarker is total C26 ceramide, preferably total C26 ceramide is cis-C26 ceramide and trans-C26 ceramide.
Embodiment 129. Use according to any one of embodiments 125 to 128, wherein the detection comprises the use of HPLC.
Embodiment 130. Use according to any one of embodiments 125 to 129, wherein the mass spectrometric analysis comprises or uses MS/MS.
Embodiment 131. Use of a biomarker for the diagnosis of cystic fibrosis, preferably in a method according to any one of embodiments 1 to 124, wherein the biomarker is selected from the group consisting of C26 ceramide, a medium ceramide and a long ceramide, wherein a medium ceramide is a ceramide of formula (2) 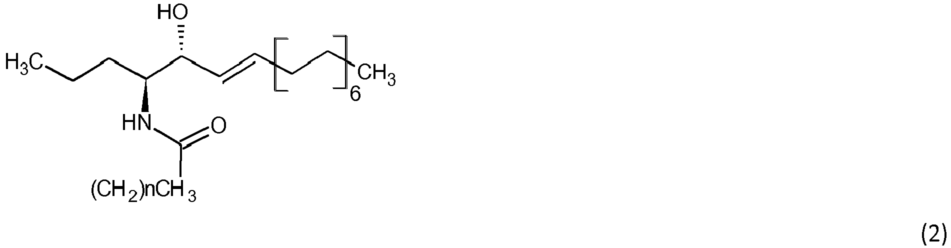 wherein n is any integer from: 8, 10, 12, 14 and 16 and
   a long ceramide is a ceramide of formula (2) 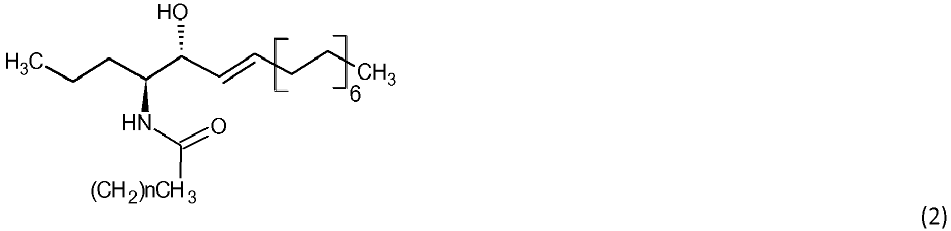 wherein n is any integer from 18, 20, 22,24, 26 and 28.
Embodiment 132. Use according to embodiment 131, wherein the biomarker is C26 ceramide.
Embodiment 133. Use according to any one of embodiments 131 to 132, wherein the biomarker is cis-C26 ceramide.
Embodiment 134. Use according to any one of embodiments 131 to 132, wherein the biomarker is total C26 ceramide, preferably total C26 ceramide is cis-C26 ceramide and trans-C26 ceramide.
Embodiment 135. A kit for determining the presence of a biomarker in a sample from a subject, wherein the kit comprises
   a) an interaction partner of the biomarker;
   b) optionally a solid support comprising at least one capture reagent attached thereto, wherein the capture reagent binds the biomarker; and
   c) instructions for using the solid support to detect the biomarker,
   wherein the biomarker is C26 ceramide, preferably cis-C26 ceramide.
Embodiment 136. The kit according to embodiment 135, wherein the kit is for
   a) use in a method for diagnosing cystic fibrosis;
   b) use in a method for determining the course of cystic fibrosis in a subject; and/or
   c) use in a method for determining the effectiveness of at least one treatment applied to a subject,
   wherein preferably the method of a), b) and/or c) is a method according to any one of embodiments 1 to 124.
Embodiment 137. Use of a compound selected from the group consisting of N-lauroyl sphingosine, lyso-Gb2, a C17 ceramide, a C19 ceramide, a C21 ceramide, a C23 ceramide and a C25 ceramide as an internal standard in
   a) use in a method for diagnosing cystic fibrosis;
   b) use in a method for determining the course of cystic fibrosis in a subject; and/or
   c) use in a method for determining the effectiveness of at least one treatment applied to a subject,
   wherein preferably the method of a), b) and/or c) is a method according to any one of embodiments 1 to 124.

The present inventors have surprisingly found that compound C26 ceramide constitutes a biomarker which allows for a method for diagnosing cystic fibrosis in a subject, more specifically diagnosing cystic fibrosis in a subject with high specificity and sensitivity using said C26 ceramide as the biomarker. In a preferred embodiment of the present invention C26 ceramide is cis-C26 ceramide. In a further embodiment of the present invention C26 ceramide is total C26 ceramide. In light of these embodiments, it is within the present invention that whenever it is referred to C26 ceramide, such reference also includes any embodiment of C26 ceramide, including cis-C26 ceramide and total C26 ceramide

The present inventors have also surprisingly found that the level of total C26 ceramide and cis-C26 ceramide in particular, determined in the sample from a subject by the methods of the present invention allows for diagnosing cystic fibrosis with high sensitivity and high specificity.

Furthermore, the present inventors have also surprisingly found that total C26 ceramide and cis-C26 ceramide in particular, which can be detected by the methods of the present invention, is circulating in the blood of a subject. Moreover, the present inventors have surprisingly found that total C26 ceramide and cis-C26 ceramide in particular, which is present in the blood of a subject is useful in a method for diagnosing cystic fibrosis in a subject comprising a step of detecting a biomarker in a sample from the subject, wherein the biomarker is total C26 ceramide and cis-C26 ceramide in particular. The present inventors have also surprisingly found that the level of total C26 ceramide and cis-C26 ceramide in particular determined in the sample from a subject by the methods of the present invention allows for diagnosing cystic fibrosis with high sensitivity and high specificity.

The present inventors also have surprisingly found that total C26 ceramide and cis-C26 ceramide is a useful biomarker contained in blood and any blood product derived therefrom such as plasma, serum or dried blood spots, for use in any of the methods and uses disclosed herein.

Finally, the present inventors have surprisingly found that the total concentration of C26 ceramide in a sample from a subject, which is preferably the sum of cis-C26 ceramide and trans-C26 ceramide in said sample, allows distinguishing a subject being carrier of cystic fibrosis from a subject being a non-carrier of cystic fibrosis, preferably such non-carrier of cystic fibrosis is a healthy subject.

In connection with the instant invention it is referred to the concentration or level of C26 ceramide. Such concentration or level of C26 ceramide is preferably determined as follows. In the analytical set-up as described in the example part in more detail an internal standard is added to the sample to be analyzed. In the course of such analysis a chromatogram is obtained indicating as individual peaks the various compounds detected in the sample. The various compounds include, among others, C26 ceramide and the internal standard. In order to determine from such chromatogram and the peaks indicated therein the concentration or level of C26 ceramide the peak area of the peak corresponding to C26 ceramide and the peak area of the peak corresponding to the internal standard is determined. The concentration of C26 ceramide is obtained using a standard curve of the C26 ceramide at different concentrations in the presence of internal standard at known concentration. In those embodiments of the methods of the present invention where the concentration or level of C26 ceramide is used, the concentration of C26 ceramide is preferably the normalized concentration of C26 ceramide.

Cystic fibrosis is a genetic disease inherited in an autosomal recessive manner. It is caused by the presence of mutations in both copies of the gene for the cystic fibrosis transmembrane conductance regulator (CFTR) protein. The mechanism(s) underlying ceramide accumulation in CF are complex, but in part are due to alterations in lung pH that favor high activity of the ceramide producing enzyme, acid sphingomyelinase (ASM), and low activity of the ceramide clearing enzyme, acid ceramidase (AC). Previous studies suggested that a defect in the cystic fibrosis transmembrane regulator protein (CFTR) may itself be responsible for high ceramide levels in cystic fibrosis patients High lung ceramide levels amplify the inflammatory pathology in cystic fibrosis patients and directly drive susceptibility to pathogens. Healthy individuals maintain low levels of ceramide in their lung epithelial cells through the regulated activities of the enzymes ASM and AC. In cystic fibrosis lungs ceramide levels become high due, in part, to alterations of these enzymatic activities, typically high ASM and low AC. In addition, mutations in CFTR may alter delivery of the protein to the membrane rafts which also contributes to ceramide elevation.

Mutations of CFTR protein associated with cystic fibrosis and of CFTR mutations tested in the diagnosis of cystic fibrosis are known to the person skilled in the art and can be retrieved from scientific papers using routine measures.

Ceramides are a family of lipid molecules. A ceramide is composed of sphingosine and a fatty acid. Ceramides are found in high concentrations within the cell membrane of cells. They are one of the component lipids that make up sphingomyelin, one of the major lipids in the lipid bilayer membrane of the cells. Ceramides and other sphingolipids found in cell membrane are not purely structural elements, they can participate in a variety of cellular signaling: examples include regulating differentiation, proliferation, and programmed cell death of cells. A typical ceramide has the formula: 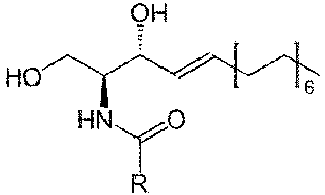 wherein R indicates the alkyl portion of the fatty acid.

C26 ceramide, which is also referred to herein as ceramide C26, is a compound of formula (1):

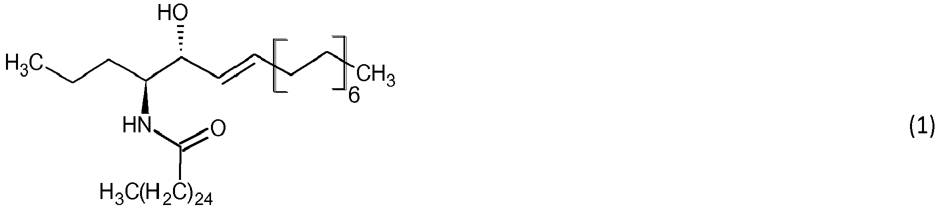

Compound C26 ceramide, which is used as a biomarker according to the present invention, can be detected, among others, by the methods disclosed herein. C26 ceramide has a molecular weight of 678, detected as MRM transition in positive mode 679 m/z to 264.4 m/z.

Apart from C26 ceramide, medium ceramides as well as long ceramides may be used as a biomarker in the methods and uses of the invention. As preferably used herein, a medium ceramide is a ceramide of formula (2) 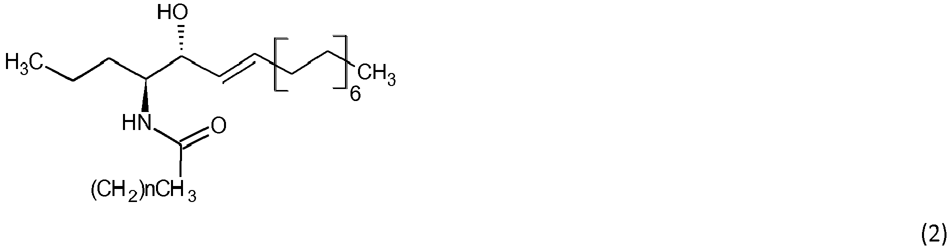 wherein n is any integer from: 8, 10, 12, 14 and 16.

As preferably used herein, a long ceramide is a ceramide of formula (2), wherein n is any integer from 18, 20, 22, 24, 26 and 28.

The term "sample" as used herein means preferably a limited quantity of a subject's material, wherein said subject's material is part of or has been taken from a subject and/or a subject's body. Preferably, said material is selected from the group comprising body fluids such as blood, a blood product, urine, saliva, cerebrospinal fluid and lymph, as well as stool or any kind of tissue and or cell material being part of a subject and/or a subject's body. It will be acknowledged by a person skilled in the art that the presence of and/or a level of a biomarker of the invention in said sample is intended to be similar to and represent the presence and/or the level of the biomarker in a larger amount of that subject's material. More precisely and as an illustrative, non-limiting example, a level of a biomarker of the invention determined in a sample of, e.g., some ml of blood from a subject also represents a level of said biomarker in the blood of the subject's body. Furthermore, in an embodiment of the method of the invention for diagnosing cystic fibrosis in a subject, a sample from the subject comprises said subject's material in a form, for example processed, fixed and/or preserved such that said sample is suitable for use in the method of the invention, whereby such processing, fixing and/or preserving preferably does not generate C26 ceramide which was not as such present in the blood of the patient. The subject's material in the sample may thus be diluted, for example with a solvent suitable for the method of the invention such as methanol and/or water, may be dried, for example on a filter card, may be resolved after having been dried such, for example with a solvent suitable for the method of the invention such as methanol and/or water, or a substance may be added, wherein said substance prevents blood from coagulation such as for example EDTA or heparin. It will be further understood by a person skilled in the art that it is within the present invention and its various aspects and embodiments that said subject's material is separated into single components of said subject's material and/or single components of said subject's material are extracted from said subject's material, for example blood is separated into plasma or serum and cellular blood components or protein is precipitated from the sample. Accordingly, in an embodiment of any method according to the present invention wherein the method comprises protein precipitation and/or HPLC, precipitation of protein preferably results in a) a precipitation of cellular blood components and/or protein, more preferably forming a pellet after a step of centrifugation, and b) the biomarker being preferably not precipitated and being present in the supernatant after a step of centrifugation. A person skilled in the art will immediately understand that in an embodiment of any method according to the present invention wherein the method comprises HPLC a supernatant containing the biomarker(s) of the present invention or a part thereof is subjected to HPLC. In connection therewith it is important to understand that the supernatant or a part thereof which is subjected to HPLC comprises the biomarker to be detected as well as, preferably, an internal standard. In an embodiment of the method of the invention wherein an internal standard is added to the sample, the internal standard may be added to the sample before or after a precipitation step, i.e. the internal standard may be added into the sample immediately after the sample is taken from the subject, or may be added to the supernatant which is subjected to HPLC, as well as in between these points in time. A person skilled in the art will know, how and when an internal standard is preferably added to the sample in order to achieve an accurate detection and determination of a level of the biomarker.

It will be immediately understood that after such processing, fixing and/or preserving the sample is subjected to the methods of the invention for detecting and/or determining a or the level of a biomarker contained in said sample, whereby such processing, fixing and/or preserving preferably does not generate C26 ceramide which was not present in the sample from the patient as such.

In an embodiment of the methods of the present invention a sample as used in such methods is prepared from a primary such as whole blood. In an embodiment of the various aspects of the invention the primary sample is whole blood which is, in an embodiment, processed such that it is collected on a dry blood filter card; preferably approximately 3µl of full blood are collected on a spot of said dry blood filter card having a diameter of 3 mm. A person skilled in the art will acknowledge that the exact volume thus collected may vary depending on the hematocrit of the specific patient.

As particularly evident from the example part the present invention provides methods for the diagnosis of cystic fibrosis and biomarkers used in said methods which allow the diagnosis of cystic fibrosis with high sensitivity and high specificity suitable of clinical application.

The term "cystic fibrosis status" as used herein, preferably refers to the status of the disease in the subject. Examples of cystic fibrosis disease statuses include, but are not limited to, the subject's risk of suffering or developing cystic fibrosis, the stage of the disease in a subject and the effectiveness of treatment of the disease. Other statuses and degrees of each status are known in the art. In an embodiment of the present invention the cystic fibrosis status comprises a severe, mild, or healthy cystic fibrosis status.

The term "diagnosing" as used herein, preferably means determining the presence or the absence of a disease or disorder in a subject and/or determining whether a subject is at risk for developing a disease, a disorder or symptoms related to a disease or disorder as well as predicting a status of a disease. "Diagnosis" or "diagnosing" as used herein also preferably means that a cause of symptoms of a disease which are present or will be present is identified.

In connection therewith it is important to note that a person skilled in the art, such as a skilled clinician consulted by a subject suffering from symptoms or suspected to be ill, applies the methods of the present invention or makes the methods of the present invention applied and thus determines whether a subject is at risk for developing a disease, particularly cystic fibrosis, whether a subject suffers from such disease or predicts the status of such disease, preferably based on the result obtained by the practicing of the methods of the present invention.

Based on said diagnosis the person skilled in the art will recommend to apply, maintain, reduce, elevate or not apply a therapy or to perform further diagnostic tests.

It is thus an embodiment of the method of the present invention for diagnosing cystic fibrosis that the method comprises giving a recommendation whether a therapy should be applied, maintained, reduced, elevated or not applied.

The term "differentially diagnosing" as used herein in connection with the method of the present invention preferably means that the method allows determining the presence or the absence of a disease or disorder in a subject and/or determining whether a subject is at risk for developing a disease, a disorder or symptoms related to a disease or disorder as well as predicting a status of a disease, wherein the disease is cystic fibrosis.

The term "detecting" in the context of the present invention means methods which include detecting the presence or absence of a substance in a sample and/or qualifying the type of said substance. Detecting can be accomplished by methods known in the art and those further described herein, including, but not limited to, the direct measurement of the affected protein(s) e.g. the sequencing of the CFTR gene. It will, however, be acknowledged by a person skilled in the art that cystic fibrosis is typically characterized by CFTR protein deficiency. Any suitable method can be used to detect one or more of the biomarkers described herein. These methods include, without limitation, mass spectrometry such as, e.g. HPLC-MS/MS, fluorescence such as, e.g., sandwich immunoassay, HPLC-fluorescence or HPLC-UV.

A biomarker as used herein, preferably is any biological compound, such as a protein and a fragment thereof, a peptide, a polypeptide, a proteoglycan, a glycoprotein, a lipoprotein, a carbohydrate, a lipid, a nucleic acid, an organic or inorganic chemical, a natural polymer, and a small molecule, which is differentially present in a sample from a subject of one phenotypic status (e.g. having a disease) as compared with another phenotypic status (e.g. not having the disease) and which may be isolated from, or measured in the sample from the subject. In an embodiment of the present invention the biomarker is different from an enzyme, preferably different from acid sphingomyelinase (ASM) and more preferably different from human acid sphingomyelinase, or a part thereof, and/or different from acid ceramidase (AC) and more preferably human acid ceramidase, or a part thereof. In an alternative embodiment of the present invention the biomarker is an enzyme, preferably an acid sphingomyelinase, preferably a human acid sphingomyelinase, or a part thereof, and/or a ceramidase and more preferably a human acid ceramidase, or a part thereof. Furthermore, the biomarker can be the entire intact molecule, or it can be a portion thereof which is preferably detected by mass spectrometric analysis, an antibody, another protein specifically binding the biomarker, functional nucleic acids specifically binding the biomarker and/or a fluorescent label. A biomarker is furthermore considered to be informative if a measurable aspect of the biomarker is associated with a given status of the patient, such as a particular status of cystic fibrosis. Such a measurable aspect may include, for example, the presence, absence, or the level of the biomarker in the sample from the subject and/or its presence as part of a profile of biomarkers. A profile of biomarkers comprises at least two such measurable aspects, where the measurable aspects can correspond to the same or different classes of biomarkers such as, for example, a nucleic acid and a carbohydrate. A biomarker profile may also comprise at least three, four, five, 10, 20, 30 or more measurable aspects. In one embodiment, a biomarker profile comprises hundreds, or even thousands, of measurable aspects. In another embodiment, the biomarker profile comprises at least one measurable aspect of at least one biomarker and at least one measurable aspect of at least one internal standard.

In an embodiment of any method according to the present invention an internal standard is added to a sample from a or the subject. It will be acknowledged that by said addition of internal standard, also referred to herein as IS, to the sample, i.e. spiking of the sample, to be subjected to such method according to the present invention, the concentration of IS in the sample is known and, e.g., by determining the area under the peak, i.e. the peak area, of the internal standard in, e.g., an HPLC-mass spectrometric chromatogram the relation between a peak area and a concentration of a substance, e.g. of IS, and/or the biomarker of the present invention, i.e. C26 ceramide, is established and thus a means provided for determining the level of the biomarker in the sample. A person skilled in the art will further acknowledge that various molecules may be used as an IS. Nevertheless an IS having a similar chemical structure compared to the molecule such as the biomarker, e.g. a ceramide or ceramide derivative, is preferable. In a preferred embodiment the molecule being the IS can be distinguished from the biomarker of the present invention, e.g. C26 ceramide, in the methods of the present invention. In a further preferred embodiment the IS is selected such that a molecule which is ideally not present or rare in nature. In accordance therewith, the present inventors have in an embodiment chosen C25 ceramide as an internal standard which is not present as such in nature. C25 ceramide is of the following structure:

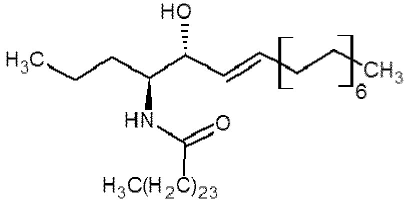

Other compounds which are useful as internal standard in the practicing of the methods of the invention include, but are not limited to,

N-lauroyl sphingosine of the following formula 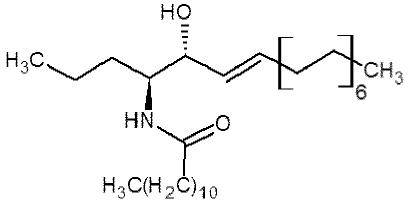 lyso-Gb2 of the following formula 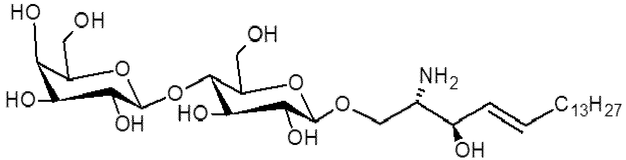 C17 ceramide of the following formula 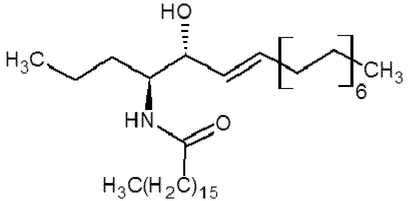 C19 ceramide of the following formula 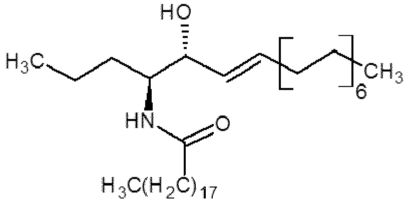 C21 ceramide of the following formula 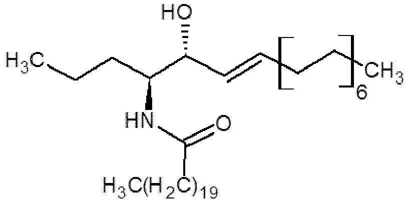 C23 ceramide of the following formula 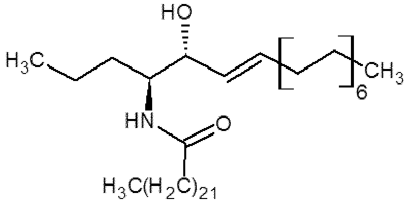 and C27 ceramide of the following formula

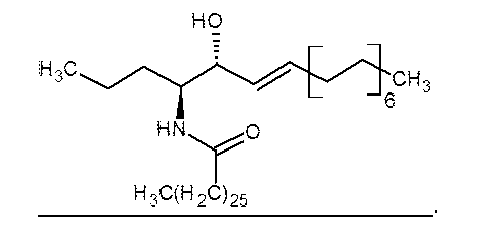

In an embodiment of the various aspects of the present invention where the internal standard is added to a sample from a subject, it is preferred that the IS is added such that it is dissolved in a solvent, e.g. *ethanol,* prior to said addition to the sample. In a further preferred embodiment that the solvent is selected such that said solvent is capable of causing protein precipitation, preferably is capable of causing the protein precipitation step as subject to the method of the present invention.

In some embodiments of the methods of the present invention a protein precipitation and/or protein precipitation step is part of the methods of the present invention. It will be understood that precipitation as used herein, preferably means the formation of a solid in a solution, i.e. for example the formation of a protein precipitate in a sample, e.g. serum, from a subject. When precipitation, e.g. protein precipitation, occurs in a sample, the solid formed is called the precipitate, or when compacted by a centrifuge, a pellet. The liquid remaining above the solid is in either case called the supernatant. The present invention contemplates different methods of precipitation and/or separating said supernatant and said precipitate or pellet, comprising, among others, settling or sedimentation and centrifugation. A person skilled in the art will know further methods for protein precipitation and/or for separating a supernatant and a protein precipitate, nevertheless said skilled person will acknowledge that if a method, preferably a method of the invention, is applied where precipitated protein will disable a device such as a column or HPLC-column used in connection with the present invention the precipitated protein is preferably separated from the solvent and/or the sample.

In some embodiments of the methods of the present invention a level of a biomarker of the present invention is compared to a level of the same or another biomarker of the present invention determined in another sample, e.g. from the same patient, from another patient, from a control and/or from the same or different points in time, and/or a cut-off value, and/or a level of a control and/or a level of an IS. In connection therewith "comparing" or "compared to" as used herein, preferably means the mathematical comparison of the two or more values of the levels of the biomarker(s). It will thus be immediately evident whether one of said values is higher, lower or identical if at least two of such values are compared with each other.

The term "cut-off value" as used herein preferably refers to a level, concentration and/or a titer of a biomarker of the present invention. In some embodiments where a ratio of two levels, concentrations and/or titers of the biomarkers of the present invention is considered said cut-off value is referred to a value of a ratio to which the ratio of two levels, concentrations and/or titers of the biomarkers is compared, and wherein if said ratio of two levels, concentrations and/or titers of the biomarkers of the present invention determined in the practicing of the methods of the present invention is elevated, increased or higher compared to the cut-off value to which the ratio of two levels, concentrations and/or titers of the biomarkers is compared, this is indicative that the subject is suffering from or is at risk for developing cystic fibrosis. In one particular embodiment thereof using C26 ceramide as the biomarker allows for diagnosing cystic fibrosis in dried blood spots using a cut-off value for C26 ceramide of 69 nmol/L, preferably 69.1 nmol/L blood in case the biomarker is total C26 ceramide, i.e. both or the sum of cis-C26 ceramide and trans-C26 ceramide, and is 27.7 nmol/L in case the biomarker is cis-C26 ceramide. It will be acknowledged by a person skilled in the art that the cut-off value and the above cut-off values in particularly depend on the method applied when determining the same. The method used in determining said cut-off values is the one described in the Example part of the instant application and Example 1 thereof in particular the disclosure of which is incorporated into this general part of the description by reference. As preferably used herein, the cut-off value is set at the mean value of a cohort of healthy individuals plus 2x standard deviation.

In some embodiments of the methods of the present invention the level of the biomarker is also determined in a control. As used herein, a control is preferably a sample from a subject, wherein the cystic fibrosis status of said subject is known. In an embodiment a control is a sample of a healthy patient. In a further embodiment an amount of said biomarker is added to said sample of a healthy patient prior to determining the level of said biomarker in said sample of a healthy patient comprising said added biomarker, preferably in the practicing of a method of the present invention. In a further embodiment the control is a sample from at least one subject having a known cystic fibrosis status, e.g. a control patient, and in a still further preferred embodiment also comprises the genetic status with regard to mutations of the gene, affected in said disease, comprising CFTR protein, i.e. comprising the subject having homozygous and/or compound heterozygous mutations, the subject being a carrier of a mutation. In a further preferred embodiment the control is a sample from a subject not being treated for cystic fibrosis. In a still further preferred embodiment the control is a sample from a single subject or a pool of samples from different subjects and/or samples taken from the subject(s) at different points in time.

The term "level" or "level of a biomarker" as used herein, preferably means the concentration of a substance and/or titer of a substance, preferably of a biomarker of the invention and more preferably of C26 ceramide, within a sample of a subject. It will be understood by a skilled person that in certain embodiments of the methods of the invention said sample is used as such in the practicing of said methods. It is, however, also within the present invention that said sample is subject to processing prior or during its use in the methods of the present invention. In an embodiment said sample may be subjected, e.g., to a step of protein precipitation, separation, e.g., centrifugation and/or HPLC and subsequently subjected to a step of determining the level of the biomarker, e.g., using mass spectrometric analysis. It should be further noted that whenever the term "a" level of a biomarker is used in connection with a level of the biomarker of the invention which is to be determined according to or in the practicing of the present invention, "the" level of the biomarker of the present invention which is to be determined by the methods of to the present invention and which is contained in the sample subjected to the method(s) of the invention is meant.

The level of a biomarker is different between different statuses of cystic fibrosis, if the mean or median level of the biomarker in the different groups is calculated to be statistically significant. Biomarkers, alone or in combination, provide measures of relative risk that a subject belongs to one phenotypic status or another. Therefore, a biomarker of the present invention is useful in an embodiment of the present invention as markers for disease, or for therapeutic effectiveness of a drug or a treatment.

The term "determining the level" of a biomarker as used herein, preferably means methods which include quantifying an amount of at least one substance in a sample from a subject and/or quantifying an amount of said substance contained in a part of the body of the subject, such as saliva, blood, lymph, serum, plasma or liquor and/or quantifying an amount of said substance in the subject, preferably the substance being selected from the group comprising a biomarker.

It will be understood by a person skilled in the art that detecting and/or determining the level of C26 ceramide in a sample from the subject, not chemically converted, transformed or derivatized. In a further preferred embodiment a step of detecting and/or determining the level of a biomarker in a sample from the subject, wherein the biomarker is C26 ceramide, is performed subsequent to separation using HPLC by application of mass spectrometric analysis.

A subject is considered to be a healthy subject with regard to cystic fibrosis, if the subject does not suffer from symptoms associated with cystic fibrosis. In an embodiment of the methods of the invention a subject will be considered to be healthy regarding cystic fibrosis, if it has no mutation of the functional parts of the CFTR gene resulting in a reduction of or deficiency of the respective protein or the activity thereof, resulting in symptoms associated with cystic fibrosis.

In connection with the present invention and more specifically therewith a "patient" is a person presenting one homozygous mutation or multiple heterozygous mutations of the CFRT gene resulting in reduction or deficiency of the respective protein and /or protein activity, resulting in symptoms associated with cystic fibrosis. Furthermore, in connection therewith a "carrier" is a person presenting one heterozygous mutation of the CFRT gene resulting or not resulting in reduction or deficiency of the respective protein and /or protein activity, usually or preferably not resulting in symptoms associated with cystic fibrosis. In an embodiment of each and any aspect of the present invention the sensitivity of the cis-C26 ceramide used as biomarker is 100 %, in other words all patient samples can be identified. In an embodiment of each and any aspect of the present invention the specificity of the cis-C26 ceramide biomarker for cystic fibrosis patients is 99.5 % versus normal controls, preferably versus healthy individuals. In an embodiment of each and any aspect of the present invention the specificity of the cis-C26 ceramide biomarker for cystic fibrosis carriers is 83 % versus normal controls, preferably versus healthy individuals.

In an embodiment of each and any aspect of the present invention the sensitivity of the total C26 ceramide used as biomarker is 100 %, in other words all patient samples can be identified. In an embodiment of each and any aspect of the present invention the specificity of the total C26 ceramide biomarker for cystic fibrosis patients is 98.9 % versus normal controls, preferably versus healthy individuals. In an embodiment of each and any aspect of the present invention the specificity of the total C26 ceramide biomarker for cystic fibrosis carriers is 85 % versus normal controls, preferably versus healthy individuals.

Said mutations of CFTR gene will be detected if a sample from the subject is subjected to a genetic testing for such mutations as described herein. In a further embodiment of the present invention a sample from a healthy subject is used as a control sample or as a blank matrix in the methods of the present invention. A blank matrix as used herein is preferably a sample from a healthy subject. Nevertheless, it will be understood that such a blank matrix may contain a native level of C26 biomarker.

In an embodiment of the present invention the level of a biomarker is indicative for the subject for suffering from or for being at risk for developing a disease or disorder, wherein the disease or disorder is cystic fibrosis. The level of the biomarker determined in accordance with the present invention is compared to a control level of the biomarker, wherein the result of said comparison allows for diagnosing cystic fibrosis.

More specifically, comparing the level of the biomarker in the sample from the subject to the control level of the biomarker comprises comparing the level of the biomarker in the sample from the subject to a cut-off value, wherein if a level of the biomarker in the sample from the subject is elevated, increased or higher compared to the cut-off value, this is indicative that the subject is suffering from or is at risk for developing cystic fibrosis or is a cystic fibrosis carrier; and/or wherein if a level of the biomarker in the sample from the subject is decreased or lower compared to the cut-off value this is indicative that the subject is not suffering from or is not at risk for developing cystic fibrosis.

The term "being at risk for developing a disease" as used herein preferably means that it is likely that a subject will suffer from said disease and/or will develop said disease or symptoms associated with said disease, particularly if no treatment is applied. In connection therewith it has to be acknowledged that cystic fibrosis is a genetic disorder and thus the occurrence of relatives, particularly parents having said disease or having a mutation known to be the cause of said disease are indicative for a subject, e.g. the child of two cystic fibrosis patients or two cystic fibrosis carriers, to be at risk for developing said disease. It will furthermore be acknowledged that the progression of a disease is linked to the occurrence of symptoms as well as the severity of said symptoms. Accordingly, a person not suffering from symptoms at present, however, may be at risk for developing the disease, for example, because although genetically mutations of a gene, known to cause a disease are present, no symptoms or no severe symptoms occur. Nevertheless, it will be immediately understood that the methods and biomarkers of the present invention, particularly if the level of said biomarker according to the present invention is elevated, allow for diagnosing that such subject is at risk for developing the disease independent from the presence or absence of symptoms. Accordingly, the methods according to the present invention allow for determining whether a subject is at risk of suffering from cystic fibrosis. It is also within the present invention that a therapy is applied, maintained, reduced, elevated or not applied based on whether the subject is at risk of suffering from cystic fibrosis or not.

It is also within the present invention that comparing the level of the biomarker in the sample from the subject to a control level allows for determining the severity of cystic fibrosis, wherein if a level of the biomarker in the sample from the subject is elevated, increased or higher compared to the control level that is indicative that the subject is suffering from or is at risk for developing cystic fibrosis of a more severe status or progression; and wherein if a level of the biomarker in the sample from the subject is decreased or lower compared to the control level that is indicative that the subject is suffering from or is at risk for developing cystic fibrosis of a less severe status or progression. In a further embodiment of the present invention that comparing the level of the biomarker in the sample from the subject to the control level comprises comparing a level of the biomarker in said subject to a level of the biomarker detected in a sample from a control, wherein if a level of the biomarker in the sample from the subject is elevated, increased or higher compared to the control sample this is indicative that the subject is suffering from and/or is at risk for developing cystic fibrosis; and/or a level of the biomarker in the sample from the subject is elevated, increased or higher compared to the control sample this is indicative that the subject is suffering from or is at risk for developing cystic fibrosis of a more severe status or progression. Said control preferably is selected from the group comprising healthy subjects, subjects suffering from cystic fibrosis or being at risk of suffering from cystic fibrosis symptoms, subjects being positively tested for a mutation or a combination of mutations of the gene CFTR; in more preferred embodiment the control is a subject suffering from cystic fibrosis, whereby the subject suffering from cystic fibrosis is a subject different from the subject undergoing diagnosis or the same subject as the subject undergoing diagnosis with the sample examined and the sample used as a reference or control having been taken from the same subject at different points in time. In a further embodiment of the present invention a control level is determined in a sample from a control, wherein optionally C26 ceramide is added to the sample from the control in a specific quantity prior to determining the level of C26 ceramide in the sample from the control.

It is the merit of the present inventors that a method for diagnosing cystic fibrosis in a subject could be established wherein the method comprises detecting a biomarker in a sample from a subject, wherein, preferably, the biomarker is C26 ceramide, preferably further comprising determining a level of the biomarker in the sample from the subject, and more preferably further comprising comparing the level of the biomarker in the sample from the subject to a cut-off value, which shows high sensitivity. In other words, the sensitivity, which means the proportion of actual positives which are correctly identified as such, is high, which means that the percentage of cystic fibrosis patients correctly identified as having the disease is as high as has been outlined above. In contrast, in a statistic test as described herein specificity means the proportion of negatives which are correctly identified as negatives, in other words the percentage of healthy patients correctly identified as not having cystic fibrosis. A person skilled in the art will acknowledge that thus an optimal prediction of a diagnostic test such as in some embodiments of the methods according to the present invention in general aims to achieve 100% sensitivity, i.e. predict all patients having a disease, such as cystic fibrosis or being at risk of suffering from said disease, as having the disease or being at risk from suffering from said disease, respectively.

In an embodiment of the methods according to the present invention a specificity of at least 95%, to 100% is preferred. In a further embodiment of the present invention of the methods according to the present invention the methods allow for diagnosing cystic fibrosis in a subject independent from a progression status of cystic fibrosis in the subject. More specifically, the methods of the present invention allow for diagnosing cystic fibrosis in a subject having an early status of cystic fibrosis as well as in a subject having an advanced or progressed status of cystic fibrosis.

A person skilled in the art will acknowledge that although the specificity and/or the sensitivity of the methods according to the present invention are as high as described above and were determined as described in the examples hereinafter, individual cases may not be excluded where a patient having cystic fibrosis will be tested false negative or where a patient not having cystic fibrosis will be tested false positive with a method of the invention. A person skilled in the art will thus immediately acknowledge that according to the methods according to the present invention, wherein a level of a biomarker is compared to a cut-off value, such comparison to said cut-off value is for use to differentially diagnose a disease and/or from a level and/or a value in a healthy subject. Having said this, it is obvious for the person skilled in the art that also according to the methods of the present invention, wherein the method is for diagnosing cystic fibrosis patient and carrier, individual cases may not be excluded where a patient having cystic fibrosis will be tested false negative or where a patient not having cystic fibrosis will be tested false positive, or where the type and/or status is diagnosed incorrectly with a method of the invention.

Therefore, it is important to note that although the sensitivity and the specificity of the method of the present invention may vary if patient collectives other than the one reported in the Example part, e.g. varying in number of patients, will be subject to the methods of the present invention, it is the firm belief of the inventors that no method known in the prior art, especially using biomarkers, will achieve a higher specificity and a higher sensitivity compared to the methods according to the present invention. This is especially true since the limit of detection of the methods of the present invention allows for determining the level of C26 ceramide in healthy subjects.

A "limit of detection" of a substance such as C26 ceramide, as used herein, preferably is a level of the substance determined by a method for determining a level of the substance, wherein a level less then or lower then said limit of detection cannot be determined by said method. It is thus immediately clear that a "cut-off value" and a "limit of detection", as used herein, are preferably not necessarily identical, although both reflect a certain level of a substance, e.g. of a biomarker of the present invention, i.e. C26 ceramide. It will be immediately understood that a cut-off value will be selected preferably such that selectivity and sensitivity of the method are as high as possible. In contrast thereto a limit of detection represents an absolute level of the biomarker of the present invention which reflects the minimum level of biomarker which can be detected with a method for determining the level of said biomarker. It is thus immediately clear that a limit of detection depends on the method for determining a level of a substance and on the substance the level of which is to be determined by the method. A skilled person will immediately understand that a high limit of detection, e.g. higher than an ideal cut-off value would possibly result in a low sensitivity of the method since the percentage of true positives that are predicted by a test to be positive also depends on whether a level of the biomarker may be determined for said true positives. In other words, if the limit of detection is higher than an ideal cut-off value, true positives having a level of the biomarker slightly higher than the cut-off value may not be distinguished from true negatives having a level of the biomarker lower than the cut-off value since no level of the biomarker may be determined for both true positives having a level of the biomarker slightly higher than the cut-off value and negatives having a level of the biomarker lower than the cut-off value. It is thus immediately clear that a low limit of detection is of advantage. It is therefore also the merit of the inventors to show that a lower limit of detection allows for a method for diagnosing cystic fibrosis in a subject comprising a step of determining a level of a biomarker present in the sample with higher selectivity and sensitivity. An "ideal cut-off value" as used herein, preferably is the cut-off value as described herein, wherein the method using said ideal cut-off value has the highest selectivity and sensitivity.

In an embodiment of the methods according to the present invention the methods comprise a step of validating said method by diagnosing a disease or disorder, preferably cystic fibrosis in a subject by the method of the present invention; a step of diagnosing the disease or disorder, preferably cystic fibrosis, in a subject by a genetic testing, comprising sequencing of a gene, preferably sequencing of a gene a mutation of which is known to the one skilled in the art to cause the disease or disorder, more preferably sequencing the CFTR gene; and comparing the results of said method and said genetic testing. A healthy subject as used herein, preferably is considered to be healthy with regard to a disease or disorder if said subject is not suffering from symptoms associated with said disease or disorder and/or if the result of a genetic testing reveals no mutations of a gene a mutation of which is known to the one skilled in the art to cause the disease or disorder. A healthy subject also is understood to be a subject being positively tested for not having cystic fibrosis. In a preferred embodiment a healthy subject is a subject not being a carrier of cystic fibrosis.

The term "qualifying cystic fibrosis status" in a subject as used herein, preferably means a classification of a subject's biomarker profile selected from the group comprising to identify or detect the presence or absence of cystic fibrosis in the subject, to predict the onset of or the risk for developing of cystic fibrosis in the subject, to determine the course of cystic fibrosis in a subject, to determine and/or predict the severity of cystic fibrosis in a subject, to determine whether a subject suffers from an early status of cystic fibrosis or an advanced or progressed status of cystic fibrosis or to determine whether a level of a biomarker in a subject has significantly changed over time.

The term "managing subject treatment" or "subject management" as used herein, preferably refers to the behavior of the clinician or physician subsequent to the determination of cystic fibrosis status. For example, if the result of the methods according to the present invention is inconclusive or there is reason that confirmation of status is necessary, the physician may order new tests, such as testing for the function of the affected proteins and/or sequencing of the CFTR gene. Alternatively, if the status indicates that treating for cystic fibrosis is appropriate, the physician may schedule the subject for treating for cystic fibrosis. Likewise, if the status is negative or if the results show that treatment has been successful, no further management may be necessary. Nevertheless a person skilled in the art will immediately acknowledge that besides gene therapy any suitable and/or effective therapy may be applied. Furthermore it is an embodiment of the present invention that managing subject treatment comprises titrating of a dose of a drug applied as a treatment for cystic fibrosis, e.g. amount of ASM inhibitor applied or administered to a patient and/or subject. In some embodiments of the methods of the present invention wherein a level of a biomarker present in a sample from a subject is determined at several points in time, or is compared to other levels of the biomarker, a cut-off value and/or a level of said biomarker in a control and/or another value of a ratio of the levels of two biomarkers, a skilled person will apply or not apply a therapy, or amend a therapy already applied in order to treat or not to treat, or to continue treating cystic fibrosis.

It is within the present invention that a skilled person will apply a dosage and/or maintain a dosage or amend a dosage, e.g. apply a dosage or a higher dosage, i.e. elevate a dosage, if such a comparison of the level of a biomarker e.g. that the level of said biomarker is higher than for example, a cut-off value, i.e. the patient is diagnosed to have cystic fibrosis; or that a level determined in the same patient earlier in time is lower or the same, i.e. a therapy applied is not sufficient, i.e. does not result in a decrease in the level of the biomarker. On the other hand, a skilled person will apply or not apply a dosage or maintain or reduce a dosage, e.g. apply no dosage or a lower dosage, i.e. decrease a dosage, if such a comparison of the level shows e.g. that the level of said biomarker is lower than for example, a cut-off value, i.e. the patient is diagnosed not to have cystic fibrosis; or that a level determined in the same patient earlier in time is higher, i.e. a therapy applied is sufficient, i.e. does result in a decrease in the level of the biomarker. Nevertheless, it will also be immediately understood that a skilled person will consider a patient's history, i.e. a skilled person managing treatment of a patient suffering from cystic fibrosis and being treated such that a level of biomarker is lower than a cut-off value, for example, will not decide to stop treatment rather than decrease a dosage and increase the time between further applications of the methods of the present invention.

The course of cystic fibrosis may be determined by the method according to the present invention by determining a level of the biomarker in the sample from the subject at different points in time in the course of the disease. It is important to note that a single application of a method for diagnosing cystic fibrosis according to the present invention allows for diagnosing cystic fibrosis and in certain embodiments comprises a step of managing subject treatment based on the diagnosis of whether the subject is suffering from or is at risk for developing cystic fibrosis. If a subject a sample of whom is thus subjected to the method of the present invention, is tested positive for suffering from or being at risk for developing cystic fibrosis a skilled clinician will know how to decide concerning managing subject treatment. It will be immediately understood that independent of the decision of a skilled clinician on how to manage subject treatment the skilled clinician may decide for at least one additional application of the method according to the present invention at a later point in time. It is thus an embodiment of the present invention that the levels of the biomarker are determined at the different points in time, wherein different points in time means at least two points in time, may be compared. Without wishing to be bound by any theory the present inventors have found that the level of the biomarker of the present invention in samples form one particular patient may be correlated to the severity of the disease in said patient at the point in time the sample from the patient is taken. It will thus immediately be understood that an elevated level of the biomarker determined in the sample of a later point in time compared to the level of the biomarker determined in the sample of an earlier point in time is indicative for a more severe status of the subject at the later point in time compared to the status of the subject at the earlier point in time. A decreased level of the biomarker and/or ratio of the levels of two biomarkers determined in the sample of a later point in time compared to the level of the biomarker determined in the sample of an earlier point in time is indicative for a less severe status of the subject at the later point in time compared to the status of the subject at the earlier point in time. Accordingly, in one aspect the present invention provides a method for determining the course of cystic fibrosis in a subject comprising the step of determining at several points in time a level of a biomarker present in a sample from the subject, wherein the biomarker is C26 ceramide. In a further aspect the invention concerns a method for determining the effectiveness of at least one treatment applied to a subject being positively tested for suffering from or being at risk for developing cystic fibrosis comprising the step of determining at several points in time a level of a biomarker present in a sample from the subject, wherein the biomarker is C26 ceramide. It will be immediately understood by a person skilled in the art that the methods of the present invention thus allow for selecting a therapy and/or adjusting the doses and/or dosage of a selected therapy based on the results of the method of the invention. If, for example, the subject is scheduled for treatment for cystic fibrosis the method for diagnosing cystic fibrosis in a subject according to the present invention may be applied every 3 months and levels of the biomarker thus determined will be compared in order to determine the effectiveness of the treatment(s) and/or therapy/therapies applied to the subject. If the subject reaches a status, wherein a stable level of the biomarker is maintained over time the frequency of application of the method for diagnosing cystic fibrosis in a subject according to the present invention may be reduced to every 6 month. If the dosage of the therapy is changed, e.g. the amount of ASM inhibitor applied to the subject are reduced or increased, the frequency of application of the method for diagnosing in a subject according to the present invention may be set back to every 3 month. By comparison of the determined levels of the biomarker in the samples from the subject the skilled physician will recognize whether the level of the biomarker increases, decreases or whether a stable level of the biomarker is maintained over time. Accordingly, the skilled physician may decide to reduce the dosage of the therapy, e.g. the amount of ASM inhibitor applied; to increase the dosage of the therapy; or to maintain the dosage of the therapy according to the comparison of the levels of the biomarker with the method according to the present invention.

A person skilled in the art thus will acknowledge that the progression, i.e. course of cystic fibrosis, as well as the effectiveness of a therapy in a single subject can be monitored by frequent determining of the level of C26 ceramide in samples from the subject.

In a further aspect the invention concerns a method for determining the effectiveness of at least one treatment applied to a subject being positively tested for suffering from or being at risk for developing cystic fibrosis, wherein the method comprises the step of determining at several points in time a level of a biomarker present in a sample from the subject, wherein the biomarker is C26 ceramide. In connection with what has been outlined above in relation to managing subject treatment a person skilled in the art will immediately understand that the effectiveness of one treatment or the combination of at least two treatments may be compared by applying the methods of the present invention. Thus, it is possible to test and compare several new drugs, dosage forms, dosages or treatments for cystic fibrosis by the method of the present invention.

It is an embodiment of the present invention that the method for diagnosing cystic fibrosis according to the present invention is independent of whether the subject has or has not been previously treated for cystic fibrosis. In accordance therewith, the sample from the subject may be a sample from a subject who has been previously treated for cystic fibrosis as well as a sample from a subject who has not been previously treated for cystic fibrosis. It is thus a further embodiment of the present invention that the methods of the present invention comprise a step of managing subject treatment and/or determining a level of the biomarker in the sample from the subject after subject management. Said subject treatment can be based on the diagnosis of whether the subject is suffering from or is at risk for developing cystic fibrosis; on the detection of the biomarker in a sample from the subject after subject management; or on the determining of the level of the biomarker in the sample from the subject after subject management, with the biomarker being C26 ceramide. Nevertheless, a person skilled in the art will understand that a sample of some patients not having cystic fibrosis or of some patients being successfully treated for cystic fibrosis will show a level of C26 ceramide lower than the limit of detection and/or the cut-off value.

Without wishing to be bound by any theory the data presented in the example part demonstrates that the level C26 ceramide present in a sample from a subject correlates with the severity of the disease in a subject suffering from cystic fibrosis, whereby preferably the severity of a given disease including cystic fibrosis is independent form the healthy control and the severity of said given disease including cystic fibrosis is determined by comparison between different patients suffering from said given disease including cystic fibrosis. In connection therewith the present inventors assume that although, in principle, the level of C26 ceramide is different in particular individuals, and more specifically may be different in particular individuals having the same mutation(s), that the higher is a level of C26 ceramide, the higher is the severity of a course of cystic fibrosis in terms of a statistical mean according to a clinical score. Thereby the level of C26 ceramide correlates with the severity of cystic fibrosis in that in patients being positively tested for distinct mutations of the CFTR gene, being known to generally cause a mild or a more severe course of cystic fibrosis, a level of C26 ceramides, determined in said patients statistically correlates with the severity generally related to such mutation.

Thus a further aspect of the present invention concerns a method for determining the severity of cystic fibrosis in a subject comprising
a) a step of determining a level of the biomarker present in a sample from the subject wherein the biomarker is C26 ceramide and
b) a step of determining the severity of cystic fibrosis, e.g. by comparing the level of C26 ceramide in a subject preferably determined by a method of the present invention to a clinical score.

The level of the biomarker will be correlated with the severity of the disease and subsequently with the type of mutation in the CFTR gene. A "mutation usually linked to a more severe course of cystic fibrosis" as used herein preferably is known to cause a more severe course of cystic fibrosis - this is especially true in case the subject is homozygous as to said mutation. Corresponding to that in an embodiment a higher mean-level of C26 ceramide is determined in the homozygous compared to the homozygous mutation usually linked to a milder course of cystic fibrosis. A person skilled in the art will know clinical scores to categorize the severity of cystic fibrosis or symptoms or a group of symptoms thereof. It is thus an embodiment of the method of the present invention that the course of cystic fibrosis in a patient is predicted and more particularly the severity of cystic fibrosis is determined based on the level of the biomarker determined according to the method of the present invention.

A person skilled in the art will acknowledge that a level of the biomarker of the present invention determined in a sample from a subject wherein said level of the biomarker is correlated with the severity of cystic fibrosis as described above, will be indicative for applying a certain therapy and/or dose or dosage of said therapy. For example, if the level of the biomarker determined according to the methods of the invention is correlated with "severe" cystic fibrosis status the subject is scheduled for treatment of cystic fibrosis and the method for diagnosing cystic fibrosis in a subject according to the present invention may be applied every 3 months and levels of the biomarker thus determined will be compared in order to determine the effectiveness of the treatment(s) and/or therapy/therapies applied to the subject. If the subject reaches a status where the level of the biomarker is correlated with a "mild" cystic fibrosis or wherein a stable level of the biomarker is maintained over time, the frequency of application of the method for diagnosing cystic fibrosis in a subject according to the present invention may be reduced to every 6 month. It will be acknowledged by a person skilled in the art that such repeated diagnosing of a subject using the method for diagnosing cystic fibrosis of the present invention, preferably in case such subject having previously been diagnosed as suffering from cystic fibrosis, is actually a monitoring of such subject and insofar, preferably in case such subject having previously been diagnosed as suffering from cystic fibrosis, the method for diagnosing of the present invention can, particularly under such conditions, also be referred to as a method of monitoring cystic fibrosis.

In another aspect the present invention is related to a method of determining the effectiveness of a composition for the treatment of cystic fibrosis. Such method may comprise the steps of determining a level of C26 ceramide in a subject having cystic fibrosis; administering to said subject said compound in an amount sufficient to determine the effectiveness of said compound; re-determining the level of C26 ceramide in said subject; comparing the level of C26 ceramide determined before and after administering said composition, wherein a lower level of C26 ceramide determined after administering said composition compared to the level of C26 ceramide determined prior to administering said composition indicates the effectiveness of said compound for treating cystic fibrosis.

It is the merit of the present inventors having found that the biomarkers of the present invention and C26 ceramide in particular are/is useful for the diagnosis of cystic fibrosis in a subject independent from the age of the subject. It is thus an embodiment of the present invention that the method of the present invention allows for diagnosing cystic fibrosis in a subject independent from age.

All of the methods disclosed in connection with the various aspects of the invention as to the diagnosis of cystic fibrosis may be equally used for prognosis of cystic fibrosis.

The present invention is now further illustrated by the following figures and examples from which further features, embodiments and advantages may be taken.

### More specifically,

Fig. 1a is a histogram indicating levels of cis-C26 ceramide in DBS of healthy controls, i.e. healthy subjects;
Fig. 1b is a histogram indicating levels of total C26 ceramide in DBS of healthy controls, i.e. healthy subjects, i.e. healthy subjects;
Fig. 2 is a diagram (whisker plot) indicating the level of cis- C26 ceramide in DBS of cystic fibrosis patients, cystic fibrosis carriers and healthy subjects; and
Fig. 3 is a diagram (whisker plot) indicating the level of total C26 ceramide in DBS of cystic fibrosis patients, cystic fibrosis carriers and healthy subjects.

### Examples

In the Examples described in the following DBS (dried blood spot) was used as a sample from a subject. Nevertheless, a person skilled in the art will acknowledge that depending on the used type of sample from a subject, e.g. comprising saliva, liquor, plasma, serum, full blood, blood on a dry blood filter card or another blood product, the method of the present invention has to be adjusted to the type of sample and furthermore a cut-off value has to be determined for each type of sample according to the method described in the following examples.

### Example 1: Method for the detection of C26 ceramide in DBS

### Equipment

For detecting C26 ceramide and/or a substance with molecular weight of 678, detected as MRM transition in positive mode 679 m/z to 264 m/z, in a sample of plasma from a subject the following equipment was used:
Apparatus / Piece of Equipment Type / Producer
UPLC Aquity Series (pumps, autosampler, column manager), Waters, UK
Mass selective detector TripleQuad 5500, AB SCIEX, Germany
Multi-tube vortexer DVX-2500 Henry Troemner LLC, USA
Vortex mixer Vortex Genie 2; Scientific Industries, USA
Centrifuge Megafuge 1.0; Heraeus, Germany
Multipette(s), pipette(s) Eppendorf, Germany
Water bath SW21-C, Julabo, Germany

### Reagents

For detecting C26 ceramide in a sample from a subject the following reagents were used. To that extent that values depend on temperature (e.g. the pH value) such values were determined at a temperature of 25°C.
Acetonitrile (ACN) HPLC-grade or Gradient
Acetone 99.5 %
Dimethylsulfoxide (DMSO) HPLC grade
Ethanol (EtOH) p.a., 96 %
Formic acid (FA) p.a., 98 - 100 %
Methanol (MeOH) Gradient (LiChrosolv)
Trifluoroacetic acid (TFA) purum > 98 %
Water ASTM-I

The abbreviation "p.a." as used herein means "pro analysis".

The term "purum" as used herein, preferably means a commercial grade of a chemical compound having a purity of the above specified value.

ASTM-I as used herein refers to a water grade standard purity achieved by purification methods comprising Reverse Osmosis and Ultraviolet (UV) Oxidation.

### Preparation of Calibration Standards

For calibration, all calibration standard C26 ceramide mentioned above having five concentration levels between 2.00 and 200 ng/mL were used.

### Preparation of Internal Standard

The Internal Standard (IS 1) stock solution was prepared dissolving 25 µg of C25 ceramide (as delivered by Avanti) in DMSO / MeOH (1/1; vol/vol) to a concentration of 50 ng/mL.

### Storing of Samples and Solutions

Control samples or study samples either were immediately stored below -20°C. Standard solutions as well as Internal Standard working solutions were stored between 2°C and 8°C until use.

### Sample Preparation for Analysis

3 punches Ø of 3.2 µm were cut from the filtercard with dried blood spots and subjected to extraction in internal standard solution in DMSO: water: ethanol vol. 1:1:2 for 60 minutes at 37°C and sonicated 20 minutes at 40°C. The solution containing internal standard and blood extract was transferred to a filter plate and then to a 96 well plate by centrifugation at 3.500 rpm.

### Methods

A person skilled in the art will acknowledge that methods for detecting C26 ceramide in a sample from a subject using mass spectrometric analysis may also employ other transitions and fragments which allow for specific detection of and/or quantification of C26 ceramide in said sample from a subject.

### Softwa re

Data acquisition, data processing, statistics and calculations were performed using Analyst software 1.6.2 or higher (AB SCIEX, USA/Canada).

### Example 2: Determining C26 ceramide in DBS from healthy subjects and cystic fibrosis patients and cystic fibrosis carriers

Using the methods outlined in Example 1, the content of cis-C26 ceramide (referred to as "Isoform 1" in Table 1) and trans-C26 ceramide (referred to as "Isoform 2" in Table 1) was determined in DBS from a total of 192 healthy subjects. Similarly, the content of cis-C26 ceramide (referred to as "Isoform 1" in Table 1) and trans-C26 ceramide (referred to as "Isoform 2" in Table 1) was determined in DBS from a total of 15 previously genetically diagnosed cystic fibrosis patients and in DBS from a total of 40 cystic fibrosis carriers (a priori genetically proven). From such data, the ratio (cis-C26 ceramide/trans-C26 ceramide), in referred to as "Ratio" in Table 1, and the total content of C26 ceramide (content of cis-C26 ceramide + content of trans-C26 ceramide), referred to as "Total" in Table 1, were determined. The cut-off value was set at the mean value of the cohort of healthy individuals plus 2x standard deviation.

The results thereof are shown in Tables 1a, 1b and 1c.

**Table 1a: Summary of C26 ceramide levels in healthy controls, cystic fibrosis patients and cystic fibrosis carriers**

| | **Ceramide C26:0 (nmol/L)** | | | | |
|---|---|---|---|---|---|
| **Samples** | **N** | **Isoform1** | **Isoform2** | **Ratio** | **Total** |
| ***Cut-off*** | | **27.7** | **-** | **-** | **69.1** |
| **Cystic Fibrosis patients** | **15** | **67.3 ± 26.0** | **39.3 ± 29.4** | **1.9 ± 1.0** | **106.5 ± 40.74** |
| **Cystic Fibrosis carriers** | **40** | **49.3 ± 52.8** | **44.9 ± 20.96** | **1.5 ± 1.1** | **93.9 ± 55.28** |
| **All Normal Controls** | **192** | **18.3 ± 4.97** | **31.6 ± 8.6** | **0.6 ± 0.2** | **49.9 ± 9.6** |

**Table 1b: C26 ceramide levels in healthy controls**

| **No** | **Healthy control description** | **C26Cer** **Isoform 1 nmol/L** | **C26Cer** **Isoform 2 nmol/L** | **ratio** | **Total C26Cer nmol/L** |
|---|---|---|---|---|---|
| **1** | **Newborn 1** | **13,1** | **51,5** | **0,3** | **64,6** |
| **2** | **Newborn 2** | **20,0** | **45,0** | **0,4** | **65,0** |
| **3** | **Newborn 3** | **15,3** | **46,4** | **0,3** | **61,7** |
| **4** | **Newborn 4** | **13,6** | **40,4** | **0,3** | **54,0** |
| **5** | **Newborn 5** | **14,2** | **22,0** | **0,6** | **36,2** |
| **6** | **Newborn 6** | **19,1** | **18,4** | **1,0** | **37,5** |
| **7** | **Newborn 7** | **18,8** | **45** | **0,4** | **63,8** |
| **8** | **Newborn 8** | **18,9** | **32,6** | **0,6** | **51,5** |
| **9** | **Newborn 9** | **16,9** | **20,1** | **0,8** | **37,0** |
| **10** | **Newborn 10** | **19,6** | **32,4** | **0,6** | **52,0** |
| **11** | **Newborn 11** | **27,0** | **19,7** | **1,4** | **46,7** |
| **12** | **Newborn 12** | **15,9** | **33,3** | **0,5** | **49,2** |
| **13** | **Newborn 13** | **16,9** | **25,1** | **0,7** | **42,0** |
| **14** | **Newborn 14** | **25,3** | **40,4** | **0,6** | **65,7** |
| **15** | **Newborn 15** | **19,3** | **32,7** | **0,6** | **52,0** |
| **16** | **Newborn 16** | **19,9** | **23,0** | **0,9** | **42,9** |
| **17** | **Newborn 17** | **19,0** | **42,8** | **0,4** | **61,8** |
| **18** | **Newborn 18** | **13,2** | **30,8** | **0,4** | **44,0** |
| **19** | **Newborn 19** | **20,9** | **45,0** | **0,4** | **65,9** |
| **20** | **Newborn 20** | **9,5** | **24,1** | **0,4** | **33,6** |
| **21** | **Newborn 21** | **6,9** | **60,7** | **0,1** | **67,6** |
| **22** | **Newborn 22** | **17,0** | **34,5** | **0,5** | **51,5** |
| **23** | **Newborn 23** | **12,6** | **40,0** | **0,3** | **52,6** |
| **24** | **Newborn 24** | **21,1** | **25,9** | **0,8** | **47,0** |
| **25** | **Newborn 25** | **27,0** | **17,4** | **1,6** | **44,4** |
| **26** | **Newborn 26** | **9,3** | **40,0** | **0,2** | **49,3** |
| **27** | **Newborn 27** | **26,0** | **27,2** | **1,0** | **53,2** |
| **28** | **Newborn 28** | **27,0** | **42,0** | **0,6** | **69,7** |
| **29** | **Newborn 29** | **25,8** | **33,5** | **0,8** | **59,3** |
| **30** | **Newborn 30** | **17,0** | **32,5** | **0,5** | **49,5** |
| **31** | **Newborn 31** | **13,0** | **42,0** | **0,3** | **55** |
| **32** | **Juvenile_0.5-4y_01** | **22,8** | **28,0** | **0,8** | **50,8** |
| **33** | **Juvenile_0.5-4y_02** | **14,0** | **16,9** | **0,8** | **30,9** |
| **34** | **Juvenile_0.5-4y_03** | **10,4** | **23,5** | **0,4** | **33,9** |
| **35** | **Juvenile_0.5-4y_04** | **22,1** | **34,2** | **0,6** | **56,3** |
| **36** | **Juvenile_0.5-4y_05** | **21,4** | **15,5** | **1,4** | **36,9** |
| **37** | **Juvenile_0.5-4y_06** | **24,6** | **13,5** | **0,9** | **38,1** |
| **38** | **Juvenile_0.5-4y_07** | **13,0** | **20,4** | **0,6** | **33,4** |
| **39** | **Juvenile_0.5-4y_08** | **24,9** | **27,8** | **0,9** | **52,7** |
| **40** | **Juvenile_0.5-4y_09** | **10,5** | **23,9** | **0,4** | **34,4** |
| **41** | **Juvenile_0.5-4y_10** | **18,4** | **36,0** | **0,5** | **54,4** |
| **42** | **Juvenile_0.5-4y_11** | **17,6** | **24,6** | **0,7** | **42,2** |
| **43** | **Juvenile_0.5-4y_12** | **17,5** | **27,3** | **0,6** | **44,8** |
| **44** | **Juvenile_0.5-4y_13** | **18,7** | **21,6** | **0,9** | **40,3** |
| **45** | **Juvenile_0.5-4y_14** | **10,9** | **20,7** | **0,5** | **31,6** |
| **46** | **Juvenile_0.5-4y_15** | **27,0** | **26,6** | **1,1** | **53,6** |
| **47** | **Juvenile_0.5-4y_16** | **17,7** | **43,6** | **0,4** | **61,3** |
| **48** | **Juvenile_0.5-4y_17** | **20,7** | **31,3** | **0,7** | **52** |
| **49** | **Juvenile_0.5-4y_18** | **19,2** | **23,6** | **0,8** | **42,8** |
| **50** | **Juvenile_0.5-4y_19** | **19,6** | **34,8** | **0,6** | **54,4** |
| **51** | **Juvenile_0.5-4y_20** | **17,4** | **24,7** | **0,7** | **42,1** |
| **52** | **Juvenile_0.5-4y_21** | **26,1** | **32,9** | **0,8** | **59** |
| **53** | **Juvenile_0.5-4y_22** | **22,6** | **29,2** | **0,8** | **51,8** |
| **54** | **Juvenile_0.5-4y_23** | **21,4** | **28,8** | **0,7** | **50,2** |
| **55** | **Juvenile_0.5-4y_24** | **23,8** | **19,1** | **1,2** | **42,9** |
| **56** | **Juvenile_0.5-4y_25** | **18,9** | **16,4** | **1,1** | **35,3** |
| **57** | **Juvenile_0.5-4y_26** | **23,0** | **31,2** | **0,7** | **54,2** |
| **58** | **Juvenile_0.5-4y_27** | **23,2** | **33,4** | **0,7** | **56,6** |
| **59** | **Juvenile_0.5-4y_28** | **27,0** | **40,4** | **0,8** | **67,4** |
| **60** | **Juvenile_0.5-4y_29** | **7,1** | **23,3** | **0,3** | **30,4** |
| **61** | **Juvenile_0.5-4y_30** | **14,4** | **46,8** | **0,3** | **61,2** |
| **62** | **Juvenile_0.5-4y_31** | **20,9** | **28,4** | **0,7** | **49,3** |
| **63** | **Juvenile_0.5-4y_32** | **8,7** | **29,5** | **0,3** | **38,2** |
| **64** | **Juvenile_0.5-4y_33** | **27,0** | **16,1** | **2,0** | **43,1** |
| **65** | **Juvenile_0.5-4y_34** | **27,0** | **31,4** | **0,9** | **58,4** |
| **66** | **Juvenile_0.5-4y_35** | **23,0** | **17,7** | **1,6** | **40,7** |
| **67** | **Juvenile_0.5-4y_36** | **26,3** | **38,5** | **0,7** | **64,8** |
| **68** | **Juvenile_4-17y_01** | **19,5** | **37,2** | **0,5** | **56,7** |
| **69** | **Juvenile_4-17y_02** | **13,3** | **36,2** | **0,4** | **49,5** |
| **70** | **Juvenile_4-17y_03** | **25,8** | **33,6** | **0,8** | **59,4** |
| **71** | **Juvenile_4-17y_04** | **22,0** | **45,0** | **0,5** | **67,0** |
| **72** | **Juvenile_4-17y_05** | **24,8** | **29,6** | **0,8** | **54,4** |
| **73** | **Juvenile_4-17y_06** | **23,6** | **36,4** | **0,6** | **60,0** |
| **74** | **Juvenile_4-17y_07** | **16,0** | **50,8** | **0,4** | **66,8** |
| **75** | **Juvenile_4-17y_08** | **23,8** | **31,6** | **0,8** | **55,4** |
| **76** | **Juvenile_4-17y_09** | **21,5** | **40,8** | **0,5** | **62,3** |
| **77** | **Juvenile_4-17y_10** | **18,4** | **43,0** | **0,3** | **61,4** |
| **78** | **Juvenile_4-17y_11** | **11,8** | **50,4** | **0,2** | **62,2** |
| **79** | **Juvenile_4-17y_12** | **19,0** | **31,3** | **0,6** | **50,3** |
| **80** | **Juvenile_4-17y_13** | **20,7** | **17,0** | **1,2** | **37,7** |
| **81** | **Juvenile_4-17y_14** | **22,9** | **35,3** | **0,6** | **58,2** |
| **82** | **Juvenile_4-17y_15** | **16,9** | **38,7** | **0,4** | **55,6** |
| **83** | **Juvenile_4-17y_16** | **16,0** | **38,5** | **0,4** | **54,5** |
| **84** | **Juvenile_4-17y_17** | **23,0** | **29,8** | **0,8** | **52,8** |
| **85** | **Juvenile_4-17y_18** | **24,8** | **38,0** | **0,7** | **62,8** |
| **86** | **Juvenile_4-17y_19** | **19,0** | **28,4** | **0,7** | **47,4** |
| **87** | **Juvenile_4-17y_20** | **13,4** | **22,5** | **0,6** | **35,9** |
| **88** | **Juvenile_4-17y_21** | **23,9** | **19,0** | **1,3** | **42,9** |
| **89** | **Juvenile_4-17y_22** | **20,9** | **39,3** | **0,5** | **60,2** |
| **90** | **Juvenile_4-17y_23** | **14,7** | **35,3** | **0,4** | **50,0** |
| **91** | **Juvenile_4-17y_24** | **10,3** | **30,0** | **0,3** | **40,3** |
| **92** | **Juvenile_4-17y_25** | **17,0** | **21,6** | **0,8** | **38,6** |
| **93** | **Juvenile_4-17y_26** | **18,4** | **35,6** | **0,5** | **54,0** |
| **94** | **Juvenile_4-17y_27** | **20,2** | **40,8** | **0,5** | **61,0** |
| **95** | **Juvenile_4-17y_28** | **26,6** | **38,9** | **0,7** | **65,5** |
| **96** | **Juvenile_4-17y_29** | **14,9** | **48,0** | **0,3** | **62,9** |
| **97** | **Juvenile_4-17y_30** | **18,2** | **31,6** | **0,6** | **49,8** |
| **98** | **Juvenile_4-17y_31** | **21,0** | **30,5** | **0,7** | **51,5** |
| **99** | **Juvenile_4-17y_32** | **25,9** | **28,0** | **0,9** | **53,9** |
| **100** | **Juvenile_4-17y_33** | **16,1** | **37,0** | **0,4** | **53,1** |
| **101** | **Juvenile_4-17y_34** | **24,4** | **27,9** | **0,9** | **52,3** |
| **102** | **Juvenile_4-17y_35** | **19,2** | **26,7** | **0,7** | **45,9** |
| **103** | **Juvenile_4-17y_36** | **24,7** | **33,2** | **0,7** | **57,9** |
| **104** | **Juvenile_4-17y_37** | **21,0** | **31,5** | **0,7** | **52,5** |
| **105** | **Juvenile_4-17y_38** | **19,1** | **38,4** | **0,5** | **57,5** |
| **106** | **Juvenile_4-17y_39** | **21,4** | **47,0** | **0,4** | **69,4** |
| **107** | **Juvenile_4-17y_40** | **14,8** | **22,3** | **0,7** | **37,1** |
| **108** | **Juvenile_4-17y_41** | **10,2** | **18,0** | **0,6** | **28,2** |
| **109** | **Juvenile_4-17y_42** | **17,2** | **17,6** | **1** | **34,8** |
| **110** | **Adult_01** | **19,3** | **46,0** | **0,4** | **65,3** |
| **111** | **Adult_02** | **13,5** | **33,2** | **0,4** | **46,7** |
| **112** | **Adult_03** | **11,3** | **26,4** | **0,4** | **37,7** |
| **113** | **Adult_04** | **15,1** | **38,6** | **0,4** | **53,7** |
| **114** | **Adult_05** | **12,3** | **32,2** | **0,4** | **44,5** |
| **115** | **Adult_06** | **21,3** | **44,0** | **0,5** | **65,3** |
| **116** | **Adult_07** | **14,5** | **48,4** | **0,3** | **62,9** |
| **117** | **Adult_08** | **14,4** | **31,0** | **0,5** | **45,4** |
| **118** | **Adult_09** | **10,9** | **29,5** | **0,4** | **40,4** |
| **119** | **Adult_10** | **22,0** | **45,0** | **0,6** | **67,0** |
| **120** | **Adult_11** | **15,3** | **37,3** | **0,4** | **52,6** |
| **121** | **Adult_12** | **14,6** | **44,4** | **0,3** | **59,0** |
| **122** | **Adult_13** | **22,3** | **35,9** | **0,6** | **58,2** |
| **123** | **Adult_14** | **18,4** | **24,3** | **0,8** | **42,7** |
| **124** | **Adult_15** | **14,9** | **21,1** | **0,7** | **36,0** |
| **125** | **Adult_16** | **15,9** | **28,0** | **0,6** | **43,9** |
| **126** | **Adult_17** | **15,3** | **25,3** | **0,6** | **40,6** |
| **127** | **Adult_18** | **25,1** | **35,1** | **0,7** | **60,2** |
| **128** | **Adult_19** | **14,7** | **42,0** | **0,3** | **56,7** |
| **129** | **Adult_20** | **17,6** | **28,7** | **0,6** | **46,3** |
| **130** | **Adult_21** | **15,4** | **24,3** | **0,6** | **39,7** |
| **131** | **Adult_22** | **24,9** | **39,6** | **0,6** | **64,5** |
| **132** | **Adult_23** | **18,1** | **26,7** | **0,7** | **44,8** |
| **133** | **Adult_24** | **17,4** | **34,7** | **0,5** | **52,1** |
| **134** | **Adult_25** | **20,6** | **31,8** | **0,6** | **52,4** |
| **135** | **Adult_26** | **15,7** | **28,4** | **0,6** | **44,1** |
| **136** | **Adult_27** | **16,5** | **29,7** | **0,6** | **46,2** |
| **137** | **Adult_28** | **13,1** | **34,2** | **0,4** | **47,3** |
| **138** | **Adult_29** | 18,5 | 27,2 | 0,7 | 45,7 |
| **139** | **Adult_30** | **22,9** | **36,5** | **0,6** | **59,4** |
| **140** | **Adult_31** | **14,8** | **28,7** | **0,5** | **43,5** |
| **141** | **Adult_32** | **11,0** | **34,6** | **0,3** | **45,6** |
| **142** | **Adult_33** | **18,8** | **30,4** | **0,6** | **49,2** |
| **143** | **Adult_34** | **17,8** | **36,8** | **0,5** | **54,6** |
| **144** | **Adult_35** | **15,9** | **40,4** | **0,4** | **56,3** |
| **145** | **Adult_36** | **16,5** | **43,2** | **0,4** | **59,7** |
| **146** | **Adult_37** | **25,2** | **36,3** | **0,7** | **61,5** |
| **147** | **Adult_38** | **15,1** | **24,1** | **0,6** | **39,2** |
| **148** | **Adult_39** | **27,9** | **36,5** | **0,8** | **64,1** |
| **149** | **Adult_40** | **17,6** | **32,1** | **0,5** | **49,7** |
| **150** | **Adult_41** | **18,3** | **21,9** | **0,8** | **40,2** |
| **151** | **Adult_42** | **16,9** | **25,9** | **0,7** | **42,8** |
| **152** | **Adult_43** | **15,4** | **19,7** | **0,8** | **35,1** |
| **153** | **Adult_44** | **19,7** | **27,0** | **0,7** | **46,7** |
| **154** | **Adult_45** | **16,1** | **28,4** | **0,6** | **44,5** |
| **155** | **Adult_46** | **14,0** | **28,7** | **0,5** | **42,7** |
| **156** | **Adult_47** | **19,4** | **25,6** | **0,8** | **45,0** |
| **157** | **Adult_48** | **16,6** | **32,1** | **0,5** | **48,7** |
| **158** | **Adult_49** | **22,6** | **34,3** | **0,7** | **56,9** |
| **159** | **Adult_50** | **19,4** | **37,0** | **0,5** | **56,4** |
| **160** | **Adult_51** | **15,0** | **32,5** | **0,5** | **47,5** |
| **161** | **Adult_52** | **13,7** | **31,8** | **0,4** | **45,5** |
| **162** | **Adult_53** | **19,2** | **23,4** | **0,8** | **42,6** |
| **163** | **Adult_54** | **15,4** | **26,5** | **0,6** | **41,9** |
| **164** | **Adult_55** | **16,1** | **18,8** | **0,9** | **34,9** |
| **165** | **Adult_56** | **26,1** | **27,7** | **0,9** | **53,8** |
| **166** | **Adult_57** | **25,7** | **31,4** | **0,8** | **57,1** |
| **167** | **Adult_58** | **14,2** | **25,8** | **0,5** | **40,0** |
| **168** | **Adult_59** | **17,7** | **32,0** | **0,6** | **49,7** |
| **169** | **Adult_60** | **19,2** | **32,6** | **0,6** | **51,8** |
| **170** | **Adult_61** | **17,8** | **28,8** | **0,6** | **46,6** |
| **171** | **Adult_62** | **15,4** | **32,7** | **0,5** | **48,1** |
| **172** | **Adult_63** | **19,5** | **32,6** | **0,6** | **52,1** |
| **173** | **Adult_64** | **13,3** | **28,3** | **0,5** | **41,6** |
| **174** | **Adult_65** | **13,7** | **31,0** | **0,4** | **44,7** |
| **175** | **Adult_66** | **21,1** | **32,9** | **0,6** | **54,0** |
| **176** | **Adult_67** | **18,9** | **33,1** | **0,6** | **52,0** |
| **177** | **Adult_68** | **14,5** | **32,7** | **0,4** | **47,2** |
| **178** | **Adult_69** | **14,2** | **21,8** | **0,7** | **36,0** |
| **179** | **Adult_70** | **22,2** | **22,9** | **1,0** | **45,1** |
| **180** | **Adult_71** | **17,7** | **31,4** | **0,6** | **49,1** |
| **181** | **Adult_72** | **17,5** | **30,0** | **0,6** | **47,5** |
| **182** | **Adult_73** | **16,9** | **33,6** | **0,5** | **50,5** |
| **183** | **Adult_74** | **27,0** | **40,0** | **0,7** | **67,0** |
| **184** | **Adult_75** | **11,7** | **24,6** | **0,5** | **36,3** |
| **185** | **Adult_76** | **12,6** | **28,9** | **0,4** | **41,5** |
| **186** | **Adult_77** | **20,4** | **39,3** | **0,5** | **59,7** |
| **187** | **Adult_78** | **19,9** | **28,3** | **0,7** | **48,2** |
| **188** | **Adult_79** | **12,6** | **40,0** | **0,3** | **52,6** |
| **189** | **Adult_80** | **15,2** | **24,2** | **0,6** | **39,4** |
| **190** | **Adult_81** | **11,1** | **27,6** | **0,4** | **38,7** |
| **191** | **Adult_82** | **16,0** | **24,9** | **0,6** | **40,9** |
| **192** | **Adult_83** | **15,6** | **25,2** | **0,6** | **40,8** |
| | **minimum** | **6,9** | **3,5** | **0,1** | **28,1** |
| | **maximum** | **27,9** | **60,7** | **2,0** | **69,7** |
| | **mean** | **18,3** | **31,6** | **0,6** | **49,9** |
| | **Standard Deviation** | **4,7** | **-** | **-** | **9,6** |
| | **Cut-off** | **27,7** | **-** | **-** | **69,1** |

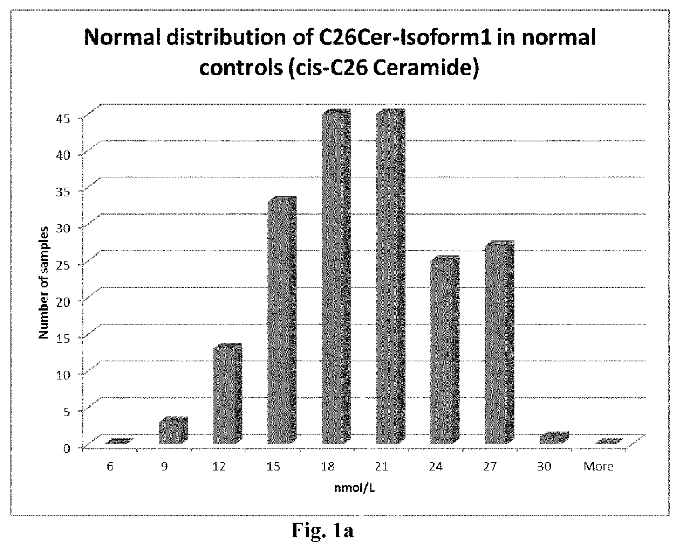

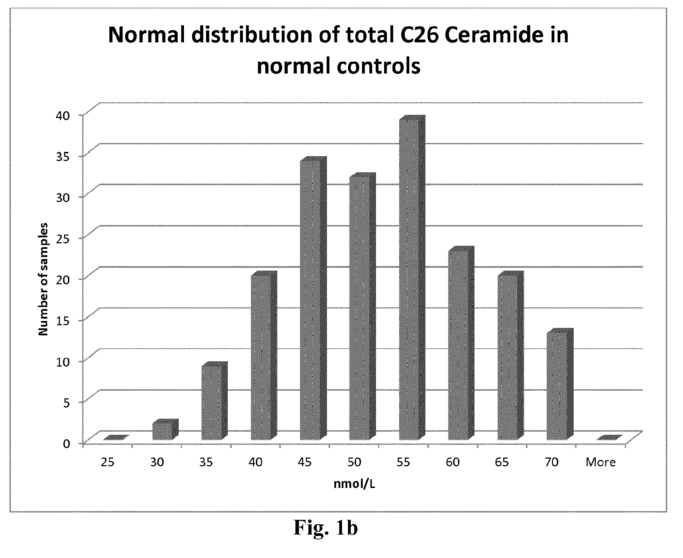

The normal distribution of cis-C26 ceramide and total C26 ceramide for healthy controls is shown in Fig. 1a and Fig 1b.

**Table 1c: C26 ceramide levels in cystic fibrosis patients and carriers**

| **No** | **Sample description** | | **C26Cer** **Isoform 1 nmol/L** | **C26Cer** **Isoform 2 nmol/L** | **ratio** | **Total C26Cer nmol/L** |
|---|---|---|---|---|---|---|
| **Cystic Fibrosis - Affected patients** | | | | | | |
| **1** | **Cystic Fibrosis _01** | **patient** | **110,7** | **61,3** | **1,8** | **171,9** |
| **2** | **Cystic Fibrosis _02** | **patient** | **53,5** | **34,2** | **1,6** | **87,7** |
| **3** | **Cystic Fibrosis _03** | **patient** | **82,8** | **52,7** | **1,6** | **135,5** |
| **4** | **Cystic Fibrosis _04** | **patient** | **78,7** | **45,7** | **1,8** | **124,4** |
| **5** | **Cystic Fibrosis _05** | **patient** | **40,7** | **58,3** | **0,7** | **99,0** |
| **6** | **Cystic Fibrosis _06** | **patient** | **55,0** | **31,2** | **1,8** | **86,2** |
| **7** | **Cystic Fibrosis _07** | **patient** | **69,7** | **19,7** | **3,6** | **89,5** |
| **8** | **Cystic Fibrosis _08** | **patient** | **49,7** | **31,6** | **1,6** | **81,3** |
| **9** | **Cystic Fibrosis _09** | **patient** | **38,8** | **31,8** | **1,2** | **70,7** |
| **10** | **Cystic Fibrosis _10** | **patient** | **47,6** | **23,3** | **2,1** | **70,9** |
| **11** | **Cystic Fibrosis _11** | **patient** | **78,3** | **47,3** | **1,7** | **125,6** |
| **12** | **Cystic Fibrosis _12** | **patient** | **57,5** | **24,9** | **2,3** | **82,4** |
| **13** | **Cystic Fibrosis _13** | **patient** | **95,4** | **27,5** | **3,5** | **122,9** |
| **14** | **Cystic Fibrosis _14** | **patient** | **64,0** | **63,0** | **1,0** | **127,0** |
| **15** | **Cystic Fibrosis _15** | **patient** | **86,3** | **36,4** | **2,5** | **122,7** |
| | **minimum** | | **38,8** | **19,7** | **0,7** | **70,7** |
| | **maximum** | | **110,7** | **63,0** | **3,6** | **171,9** |
| | **mean** | | **67,3** | **39,3** | **1,9** | **106,5** |
| | | | | | | |
| **Cystic Fibrosis - Carriers** | | | | | | |
| **1** | **CF Carrier_01** | **carrier** | **48,4** | **60,6** | **0,8** | **109,0** |
| **2** | **CF Carrier_02** | **carrier** | **97,6** | **22,2** | **4,5** | **119,7** |
| **3** | **CF Carrier_03** | **carrier** | **15,8** | **33,8** | **0,5** | **49,5** |
| **4** | **CF Carrier_04** | **carrier** | **10,9** | **55,1** | **0,2** | **66,1** |
| **5** | **CF Carrier_05** | **carrier** | **13,8** | **33,7** | **0,4** | **47,4** |
| **6** | **CF Carrier_06** | **carrier** | **28,7** | **67,9** | **0,4** | **96,6** |
| **7** | **CF Carrier_07** | **carrier** | **47,3** | **48,0** | **1,0** | **95,4** |
| **8** | **CF Carrier_08** | **carrier** | **69,2** | **49,1** | **1,8** | **107,9** |
| **9** | **CF Carrier_09** | **carrier** | **73,5** | **58,2** | **1,3** | **131,8** |
| **10** | **CF Carrier_10** | **carrier** | **58,0** | **32,7** | **1,8** | **90,7** |
| **11** | **CF Carrier_11** | **carrier** | **12,6** | **51,1** | **0,3** | **63,7** |
| **12** | **CF Carrier_12** | **carrier** | **94,5** | **31,8** | **3,1** | **126,4** |
| **13** | **CF Carrier_13** | **carrier** | **33,9** | **31,2** | **1,1** | **65,1** |
| **14** | **CF Carrier_14** | **carrier** | **92,5** | **43,1** | **2,2** | **135,6** |
| **15** | **CF Carrier_15** | **carrier** | **10,8** | **37,4** | **0,3** | **48,2** |
| **16** | **CF Carrier_16** | **carrier** | **110,5** | **38,2** | **2,9** | **148,7** |
| **17** | **CF Carrier_17** | **carrier** | **10,7** | **37,4** | **0,3** | **48,1** |
| **18** | **CF Carrier_18** | **carrier** | **13,8** | **47,9** | **0,3** | **61,7** |
| **19** | **CF Carrier_19** | **carrier** | **14,6** | **52,0** | **0,3** | **66,6** |
| **20** | **CF Carrier_20** | **carrier** | **103,9** | **64,0** | **1,7** | **167,8** |
| **21** | **CF Carrier_21** | **carrier** | **12,9** | **31,3** | **0,4** | **44,1** |
| **22** | **CF Carrier_22** | **carrier** | **16,4** | **55,7** | **0,3** | **72,2** |
| **23** | **CF Carrier_23** | **carrier** | **28,9** | **35,5** | **0,8** | **64,3** |
| **24** | **CF Carrier_24** | **carrier** | **36,6** | **34,1** | **1,1** | **70,7** |
| **25** | **CF Carrier_25** | **carrier** | **14,6** | **27,5** | **0,5** | **42,0** |
| **26** | **CF Carrier_26** | **carrier** | **60,1** | **41,3** | **1,5** | **101,4** |
| **27** | **CF Carrier_27** | **carrier** | **66,3** | **40,0** | **1,7** | **106,2** |
| **28** | **CF Carrier_28** | **carrier** | **67,1** | **28,8** | **2,3** | **95,9** |
| **29** | **CF Carrier_29** | **carrier** | **50,5** | **5,1** | **9,8** | **55,6** |
| **30** | **CF Carrier_30** | **carrier** | **89,9** | **31,8** | **2,9** | **121,7** |
| **31** | **CF Carrier_31** | **carrier** | **43,1** | **56,8** | **0,8** | **99,8** |
| **32** | **CF Carrier_32** | **carrier** | **56,1** | **56,0** | **1,0** | **112,1** |
| **33** | **CF Carrier_33** | **carrier** | **12,5** | **105,1** | **0,1** | **117,5** |
| **34** | **CF Carrier_34** | **carrier** | **19,4** | **72,2** | **0,3** | **91,6** |
| **35** | **CF Carrier_35** | **carrier** | **94,7** | **37,6** | **2,5** | **132,4** |
| **36** | **CF Carrier_36** | **carrier** | **102,5** | **78,4** | **1,3** | **181,0** |
| **37** | **CF Carrier_37** | **carrier** | **45,2** | **41,3** | **1,1** | **86,5** |
| **38** | **CF Carrier_38** | **carrier** | **131,0** | **40,1** | **3,3** | **171,2** |
| **39** | **CF Carrier_39** | **carrier** | **51,8** | **33,6** | **1,6** | **85,4** |
| **40** | **CF Carrier_40** | **carrier** | **12,0** | **47,7** | **0,3** | **59,8** |
| | **minimum** | | **10,7** | **5,1** | **0,1** | **42,0** |
| | **maximum** | | **131,0** | **105,1** | **9,8** | **181,0** |
| | **mean** | | **49,3** | **44,9** | **1,5** | **93,9** |

### Example 3: Determining the cut-off value for C26 ceramide for the diagnosis of cystic fibrosis

Based on the results presented in Examples 1 and 2, the cut-off values for the diagnosis of cystic fibrosis patients and cystic fibrosis carrier based on cis-C26 ceramide and total C26 ceramide were determined. Using dried blood spots, the cut-off value for the diagnosis of cystic fibrosis based on the level of cis-C26 ceramide is 27.7 nmol/L blood of the subject to be tested and, respectively, diagnosed, and the cut-off value for the diagnosis of cystic fibrosis based on the level of total C26 ceramide is 69.1 nmol/L blood of the subject to be tested and, respectively, diagnosed.

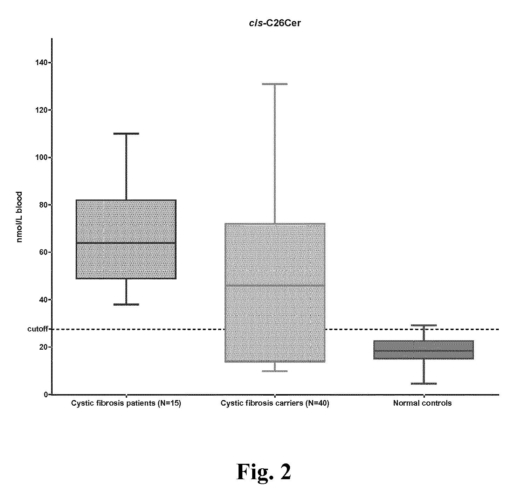

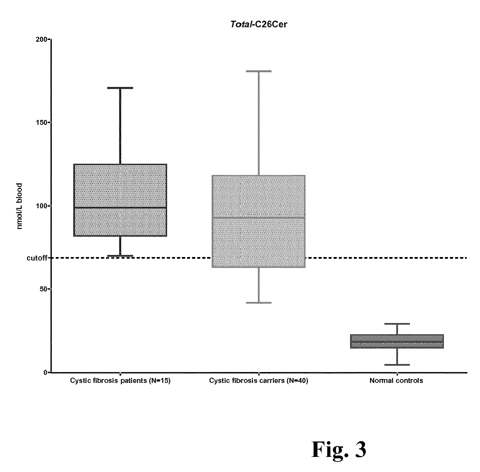

These results are also shown as a whisker plot in Figs. 2 and 3. From Said Fig. 2 it is evident that using cis-C26 ceramide as a biomarker in blood of a subject with a cut-off value of 27.7 nmol/L, cystic fibrosis patients can be distinguished from normal, i.e. healthy control subjects. From said Fig. 3 it is evident that using total C26 ceramide as a biomarker in blood of a subject with a cut-off value of 69.1 nmol/L, cystic fibrosis carriers can be distinguished from normal, i.e. healthy control subjects.

### Example 4: Positive predictive value and negative predictive value of C26 ceramide in the diagnosis of cystic fibrosis

All of the samples described in Example 1 were used for calculating the positive predictive value (PPV) and the negative predictive value (NPV) versus the healthy controls. The results are shown in Table 2.

**Table 2: PPV and NPV for C26 ceramide for the diagnosis of cystic fibrosis**

| | | Patients with Cystic fibrosis (as confirmed on molecular genetics) | | |
|---|---|---|---|---|
| | | Condition positive | Condition negative | |
| C26Cer Isoform 1 (cis-C26 ceramide) | Test outcome positive | **True positive** | **False positive** | Positive predictive value = TP / (TP + FP) = 0,937 |
| | | (TP) = 15 | (FP) = 1 | |
| | Test outcome negative | **False negative** | **True negative** | Negative predictive value = TN / (FN + TN) =1 |
| | | (FN) = 0 | (TN) = 192 | |
| | | Sensitivity = TP / (TP + FN) = 1 | Specificity = TN / (FP + TN) = 0.995 | |

| | | Patients with cystic fibrosis (as confirmed on molecular genetics) | | |
|---|---|---|---|---|
| | | Condition positive | Condition negative | |
| Total C26Cer | Test outcome positive | **True positive** | **False positive** | Positive predictive value = TP / (TP + FP) |
| | | (TP) = 15 | (FP) = 2 | |
| | Test outcome negative | **False negative** | **True negative** | Negative predictive value = TN / (FN + TN) = 1 |
| | | (FN) = 0 | (TN) = 192 | |
| | | Sensitivity = TP / (TP + FN) = 1 | Specificity = TN / (FP + TN) = 0.989 | |

The features of the present invention disclosed in the specification, the claims, the sequence listing and/or the drawings may both separately and in any combination thereof be material for realizing the invention in various forms thereof.

## Claims

1. A method for diagnosing cystic fibrosis in a subject comprising
a step a), wherein the step a) comprises detecting a biomarker in a sample from the subject, wherein the sample is a sample selected from the group consisting of blood, blood plasma, blood serum and a blood product.

2. The method according to claim 1, wherein the method comprises
a step b) wherein the step b) comprises determining a level of the biomarker present in the sample.

3. The method according to any one of claims 1 or 2, wherein the level of the biomarker is indicative whether or not the subject is suffering from cystic fibrosis or whether or not the subject is at risk of suffering from cystic fibrosis.

4. A method for diagnosing cystic fibrosis in a subject, wherein the method comprises the following steps:
i) adding an internal standard to a sample from the subject, wherein the sample from the subject is selected from the group comprising plasma, serum, blood and a blood product;
ii) optionally mixing the sample with the internal standard;
iii) subjecting the sample to a protein precipitation and/or a biomarker extraction step, whereby protein from the sample is precipitated and/or the biomarker is extracted and a first supernatant of the sample is provided;
iv) optionally subjecting the first supernatant of the sample or at least a part thereof to a first separation step which provides a second supernatant, whereby preferably the first separation step is a step of centrifugation;
v) subjecting the first supernatant and/or the second supernatant, or at least a part thereof, to a second separation step, wherein the second separation step comprises injecting at least a part of the first supernatant and/or at least a part of the second supernatant into an HPLC-MS/MS system and using an HPLC column with a gradient from acidic water to acetonitrile/acetone; wherein the HPLC column is preferably an HPLC column selected from the group comprising a C8 HPLC column and a C18 HPLC column, and wherein the second separation step provides a separated sample;
vi) subjecting the separated sample to MS/MS, wherein MS/MS comprises collision induced dissociation, in source-fragmentation, electron capture and/or transfer dissociation;
and wherein the method comprises
a step a), wherein the step a) comprises detecting a biomarker in a sample from the subject, and optionally
a step b), wherein the step b) comprises determining a level of the biomarker present in the sample, and
wherein the method is preferably a method according to any one of claims 1 to 3.

5. The method of claim 4, wherein the internal standard is selected from the group comprising N-lauroyl sphingosine, lyso-Gb2, a C17 ceramide, a C19 ceramide, a C21 ceramide, a C23 ceramide and a C25 ceramide.

6. A method of determining the effectiveness of a compound for the treatment of cystic fibrosis, wherein the method comprises the following steps:
a) determining a level of a biomarker in a sample from a subject having cystic fibrosis;
b) administering to said subject said compound;
c) determining the level of the biomarker in a sample from the subject after the compound has been administered to the subject; and
d) determining whether the level of the biomarker determined in step c) is lower than the level of the biomarker determined in step a);
wherein if a level of the biomarker determined in step c) is lower than the level of the biomarker determined in step a) this indicates the effectiveness of said compound.

7. A method for determining the course of cystic fibrosis in a subject comprising the step of determining at several points in time a level of a biomarker present in a sample from the subject.

8. The method according to any one of claims 1 to 7, wherein the biomarker is one selected from the group consisting of C26 ceramide, a medium ceramide and a long ceramide, wherein a medium ceramide is a ceramide of formula (2) 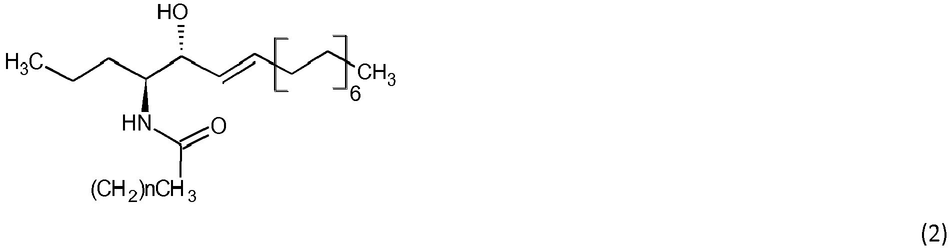 wherein n is any integer from: 8, 10, 12, 14 and 16 and
a long ceramide is a ceramide of formula (2) 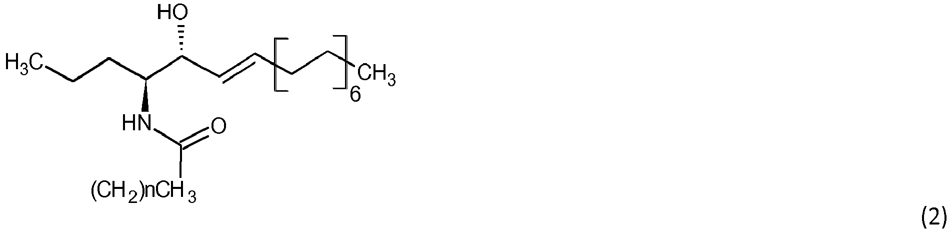 wherein n is any integer from 18, 20, 22, 24, 26, and 28.

9. The method according to claim 8, wherein the biomarker is C26 ceramide.

10. The method according to any one of claims 8 to 9, wherein the biomarker is cis-C26 ceramide.

11. The method according to any one of claims 8 to 9, wherein the biomarker is total C26 ceramide, preferably total C26 ceramide is cis-C26 ceramide plus trans-C26 ceramide.

12. The method according to any one of claims 1 to 11, wherein the biomarker is detected by means of mass spectrometric analysis.

13. The method according to any one of claims 1 to 12, wherein the subject is a human being.

14. The method according to any one of claims 1 to 5 and 8 to 13, wherein if the level of the biomarker in the sample from the subject is higher than a cut-off value this is indicative that the subject is suffering from cystic fibrosis or is at risk of suffering from cystic fibrosis and/or wherein if the level of the biomarker in the sample from the subject is lower than a cut-off value this is indicative that the subject is not suffering from or is not at risk of suffering from cystic fibrosis, preferably the cut-off value is 69 nmol/L blood, more preferably 69.1 nmol /L blood in case the biomarker is total C26 ceramide and the cut-off value is 27.7 nmol/L blood in case the biomarker is cis-C26 ceramide.

15. The method according to any one of claims 1 to 14, preferably claim 14, wherein the sample is dried blood spots (DBS).
